(19) Europäisches Patentamt
European Patent Office
Office européen des brevets

(11) Publication number: 0 217 748 B1

(12) **EUROPEAN PATENT SPECIFICATION**

(45) Date of publication of patent specification: 06.02.91 Bulletin 91/06

(21) Application number: 86810421.7

(22) Date of filing: 25.09.86

(51) Int. Cl.$^5$: C07D 487/04, C07D 487/14, C07D 471/14, C07D 495/14, C07D 498/14, C07D 513/14, C07D 493/14, A61K 31/505, A61K 31/535, A61K 31/54, // (C07D487/04, 249:00, 239:00), (C07D487/14, 249:00, 239:00, 235:00), (C07D471/14, 249:00, 239:00, 221:00), (C07D487/14, 249:00, 239:00, 209:00), (C07D495/14, 333:00, 249:00, 239:00)

(54) **2-Substituted-e-fused-[1,2,4,]triazolo-[1,5-c]pyrimidines pharmaceutical compositions and uses thereof.**

(30) Priority: 30.09.85 US 782234
20.03.86 US 841986

(43) Date of publication of application: 08.04.87 Bulletin 87/15

(45) Publication of the grant of the patent: 06.02.91 Bulletin 91/06

(84) Designated Contracting States: AT BE CH DE FR GB IT LI LU NL SE

(56) References cited:
EP-A- 0 023 773
EP-A- 0 080 176
EP-A- 0 181 282
DE-A- 2 261 095
US-A- 3 045 015
MONATSHEFTE FÜR CHEMIE, vol. 106, 1975; F.SAUTER et al.: "Synthese neuer Derivate des 2-Amino-4,5,6,7-tetrahydrobenzo[b]thiophen-3-carbonitrils: basische Substitutionsprodukte und annelierte Thieno[1,2,4]-triazole-pyrimidine", pp. 1111-1116

(73) Proprietor: CIBA-GEIGY AG
Klybeckstrasse 141
CH-4002 Basel (CH)

(72) Inventor: Francis, John E., Dr.
15 Wexford Way
Basking Ridge, NJ 07920 (US)
Inventor: Gelotte, Karl O.
106 Stanie Brae Drive
Watchung, NH 07060 (US)

EP 0 217 748 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The invention concerns e-fused [1,2,4]triazolo[1,5-c]pyrimidines, pharmaceutical compositions thereof and uses of the compounds and compositions. More particularly, the inventive compounds are compounds of the formula

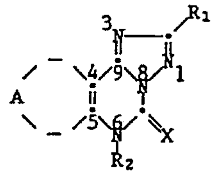

(Ia)

its tautomers and pharmaceutically acceptable salts wherein

X is O, S, NH or NR ;

R is $C_1$-$C_7$-alkyl, $C_2$-$C_7$-alkenyl, $C_2$-$C_7$-alkynyl, wherein the unsaturated bonds of the alkenyl and alkynyl groups are separated from the N atom of X by at least one saturated carbon atom, $C_5$-$C_7$-cycloalkyl or aromatic ring-$C_1$-$C_7$-alkyl wherein the aromatic ring is phenyl, naphthyl or aromatic heteroaryl with 5-7 ring members having at least one heteroatom selected from O, N and S, but not more than one O or S atom, all of the foregoing $C_1$-$C_7$-alkyl groups being optionally interrupted by a heteroatom selected from O, N or S ; or R is hydroxy, hydroxy-$C_2$-$C_4$-alkyl, phenyl, naphthyl or aromatic heteroaryl with 5-7 ring members having at least one heteroatom selected from O, N and S, but not more than one O or S atom, or the group –C(=NH)–$NH_2$ ;

$R_1$ is phenyl, naphthyl, pyridyl, thienyl, furyl, pyrrolyl, thiazolyl, isothiazolyl, oxazolyl, imidazolyl, pyrazolyl, 1,2,3-or 1,2,4-triazolyl, tetrazolyl, pyrimidinyl, quinolyl, isoquinolyl, pyrrolinyl, pyrrolidinyl, dihydro- or tetrahydrofuranyl, dihydro- or tetrahydrothienyl, pyranyl, piperidinyl, morpholinyl, pyrazolinyl, thiazolinyl, oxazolinyl, triazolinyl, or ribofuranosyl, these groups $R_1$ being unsubstituted or substituted by halogen, $C_1$-$C_7$-alkyl, halo-$C_1$-$C_7$-alkyl, hydroxy, $C_1$-$C_7$-alkoxy, hydroxy-$C_1$-$C_7$-alkyl, amino, mono- or di-$C_1$-$C_7$-alkylamino, $C_1$-$C_7$-alkoxycarbonyl, carbamoyl, or $C_1$-$C_7$-alkyl-carbamoyl ;

$R_2$ is hydrogen, $C_1$-$C_7$-alkyl, hydroxy-$C_1$-$C_7$-alkyl, $C_2$-$C_7$-alkenyl, aryl-$C_1$-$C_7$-alkyl, aryl-$C_2$-$C_7$-alkenyl or aryl wherein aryl is phenyl, naphthyl or aromatic heteroaryl with 5-7 ring members having at least one heteroatom selected from O, N and S, but not more than one O or S atom ;

A is a bivalent bridging group containing chain atoms selected from C, O, N and S which forms together with the two adjacent carbon atoms to which the chain is attached a cyclohexene, cyclopentene, piperideine, tetrahydrobenzo[b]thiophene, pyridine, cycloheptene, dihydropyrrole, isoxazole, oxazole, isothiazole, thiazole, pyrazole, oxathiazole, dithiazole, pyrrole, furan, thiophene, oxazine, thiazine, pyridazine, pyran, thiopyran, oxathiin, dioxin, dithiin, pyrazine, pyrimidine or imidazole, a benzocondensated derivative thereof, or a partially saturated benzocondensated derivative thereof, these groups A being unsubstituted or substituted by $C_1$-$C_7$-alkyl, $C_1$-$C_7$-alkoxy, hydroxy, halogen, halo-$C_1$-$C_7$-alkyl, nitro, amino, $C_1$-$C_7$-alkylthio, $C_1$-$C_7$-alkylsulfonyl, $C_1$-$C_7$-alkylsulfinyl, aryl-$C_1$-$C_7$-alkyl wherein aryl is phenyl, naphthyl or aromatic heteroaryl with 5-7 ring members having at least one heteroatom selected from O, N and S, but not more than one O or S atom, amido, carbamoyl, $C_1$-$C_7$-alkoxycarbonyl or benzoyl.

Other compounds within the scope of this invention are the related isomeric forms wherein the $R_2$ group is in another position as set forth below :

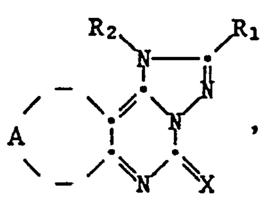 , 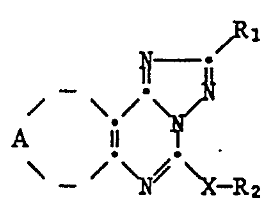 and 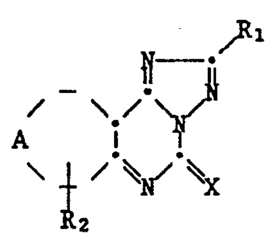

(Ib) (Ic) (Id)

The tautomeric forms represented by the formulae Ib, Ic and Id may exist together with the form represented by formula Ia.

The above compounds possess benzodiazepine agonist/antagonist and anxiomodulating effects, especially when X is oxygen. In addition, and especially those compounds wherein X is mono or substituted imino, the compounds of the invention are adenosine antagonists/agonists. The benzodiazepine antagonists are primarily useful anorectic agents, CNS stimulating agents, and agents for increasing cognitive ability. They are also useful for counteracting the sedative effects of benzodiazepine tranquillizers. The benzodiazepine agonists are primarily useful as anxiolytics, CNS depressants, and anticonvulsants. Adenosine antagonists are primarily useful as anti-asthmatic agents, and may also be used in the treatment of bradyarrhythmias associated with clinical conditions such as myocardial infarction and sleep apnea. Adenosine agonists are primarily useful as antihypertensives.

Triazolo[1,5-c]pyrimidine compounds have been described in a number of references. U.S. Patent 3,045,015 discloses primarily bicyclic compounds having an unsubstituted amino group in the 2-position of the triazolopyrimidine ring. However some tricyclic rings are generically described wherein a cyclohex-1-ene ring is fused to the [e] face of the pyrimidine ring. These compounds are claimed as bronchodilators, respiratory stimulants and antiarthritic agents. In addition, antibacterial, sedative and hypotensive properties are disclosed. In J. Med. Chem. 17, 645-8 (1974), Novinson concludes that the compounds in U.S. Patent 3,045,015 are active because they inhibit CAMP phosphodiesterase. Shishoo, in J. Heterocyclic Chem. 18, 43-6 (1981), reports on the synthesis of angular tricyclics which are triazolopyrimidines having a heterocyclic fused to the [e] face of the pyrimidine ring. These are unsubstituted at the 2-position of the triazolo pyrimidine ring system and have H, alkyl, aryl or arylalkyl in the same position wherein X is located in formula Ia.

Other heterocyclic rings fused to the [e] face of the [1,2,4]-triazolo[1,5-c]pyrimidine ring system are mentioned in Huang et al., Chem. Pharm. Bull. 22, (8) 1938-9 (1974) ; Huang at al., Tetrahedron 31, 1363-7 (1975) ; Leonard and Wiemar, J.O.C. 39, 3438-40 (1974) ; Temple et al., J.O.C. 30, 3601-3)1965) ; Sauter and Stanetty, Monatsh. Chem. 106, 111-6 (1975) ; Brown and Shinozuka, Australian J. Chem. 34, 189-194 (1981) ; Bhat, Schram and Townsend, C.A. 95, 98200z (1981) ; Bhat and Townsend, J.C.S. perkin I, 1981, 2387-2393 ; Schneller and Clough, J. Heterocyclic Chem. 1974, 975-7 ; Sangapure and Agasimundin, Indian J. Chem. B, 1980, 115-117 ; Saikachi, Matsuo and Matsuda, Yakugaku Zasshi 1969, 1434-9 ; and Petric, Tisler and Stanovnik, Monatsh. Chem. 114, 615-624 (1983). None of these compounds possess a carbonyl, thiono or imino group at the triazolopyrimidine position 7. Further, only Saikachi et al. mentions any compound having an aryl group at the 2-position of triazolopyrimidine. No biological properties are indicated.

Since the only known compounds which have (beneficial) biological activity have the 2-position of the triazolopyrimidine ring system substituted with an unsubstituted amino group, it would be expected that such a group would be indispensable for useful biological properties to be present. Surprisingly it has now been found that replacement of that amino group with an aryl, heteroaryl, or non-aromatic heterocyclyl moiety yields biologically active compounds as well. The novel compounds of the invention are useful as anxiomodulators and benzodiazepine and/or adenosine agonists/antagonists.

The compounds of the invention all have one of the structures Ia-Id set forth below (The ring numbering, unless stated otherwise, will be that of the triazolopyrimidine bicyclic ring structure set forth earlier.) :

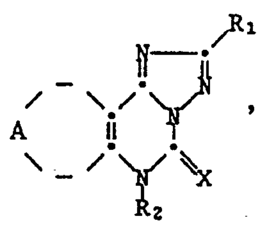
(Ia)
,
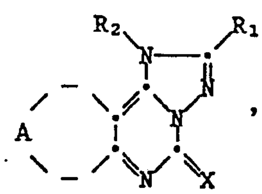
(Ib)
,
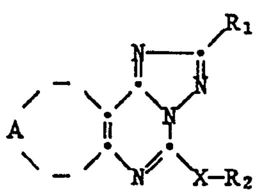
(Ic)
and
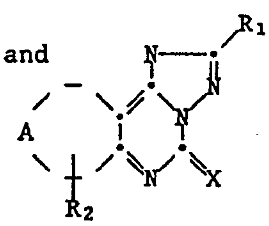
(Id)

In the compounds according to these formulae the groups X, $R_1$,$R_2$ and A have the following preferred meanings :

X is selected from O, S, NH and N-R ;

R is selected from lower alkyl, lower alkenyl, lower alkynyl, cyclo-lower alkyl, aromatic ring-lower alkyl, all of the alkyl groups being optionally interrupted by a heteroatom selected from O, N and S ; an aromatic ring ; and the group $-C(=NH)-NH_2$ ;

$R_1$ is selected from carbocyclic aromatic groups and partially saturated, totally saturated or aromatic heterocyclic groups, all of which are optionally substituted with a group selected from halogen, lower alkyl, halo (preferably fluoro) substituted lower alkyl (most preferably trifluoromethyl), hydroxy, lower alkoxy, hydroxy-lower alkyl, amino, mono-lower alkylamino, di-lower alkylamino (which alkyl groups may be the same or different), carbo-lower alkoxy, carbamoyl and lower alkylcarbamoyl ; and

$R_2$ is selected from hydrogen, lower alkyl, hydroxy-lower alkyl, lower alkenyl, aryl-lower alkyl, aryl-lower alkenyl and aryl ; preferably hydrogen.

A is a bivalent group of atoms selected from C, N, O and S, which form together with the two adjacent carbon atoms to which they are attached an optionally substituted, mono- or bicyclic aliphatic carbocyclic, or a heterocyclic ring or an optionally substituted mono- or bicyclic heteroaromatic ring, the optional substituents of which are selected from lower alkyl, lower alkoxy, hydroxy, halogen, halo (preferably fluoro)-lower alkyl, lower alkylthio, lower alkylsulfonyl, lower alkylsulfinyl, aryl-lower alkyl, amido, carbonyl, carbo-lower alkoxy and aroyl ; and pharmaceutically acceptable salts thereof.

As used within this application :

The term "lower alkyl" means alkyl having 1-7 carbon atoms, preferably 1-5 carbon atoms, and most preferably 1-4 carbon atoms ; "lower alkenyl" is alkenyl having 2-7 carbon atoms, preferably 2-5 carbon atoms, and most preferably 2-4 carbon atoms ; "lower alkynyl" is alkynyl having 2-7 carbon atoms, preferably 2-5 carbon atoms, and most preferably 2-4 carbon atoms ; and "cyclo-lower alkyl" is cycloalkyl having 5, 6 or 7 ring members.

The aromatic ring, as used in the definition of R, is phenyl, naphthyl or aromatic heterocyclyl having 5-7 ring members, preferably 6 members, containing at least one heteroatom selected from O, N and S, preferably N, but not more than one O or S atom. Preferably, aromatic rings within the scope of R are 5 or 6 membered, being carbocyclic or having 1-4 nitrogen atoms, one sulfur or oxygen atom or 1-2 nitrogen heteroatoms and optionally an oxygen or a sulfur heteroatom. Such heterocyclic groups more preferably have 1-4 nitrogen atoms, 1 oxygen atom, 1 sulfur atom, or 1-2 nitrogen atoms together with either 1 oxygen atom or 1 sulfur atom. Preferred aromatics defined by R may be selected from phenyl, pyridyl, thienyl, furyl, pyrrolyl, tetrazolyl, pyrimidinyl and fused rings thereof wherein each of the fused ring independently meets the above limitations.

Although any of the groups set forth above are suitable, R is preferably lower alkyl. Also preferred are the compounds wherein X is NH.

Aromatic rings and partially and totally saturated heterocyclic groups within the scope of $R_1$ are phenyl, naphthyl, pyridyl, thienyl, furyl, pyrrolyl, thiazolyl, isothiazolyl, oxazolyl, imidazolyl, pyrazolyl, 1,2,3- and 1,2,4-triazolyl, tetrazolyl, pyrimidinyl, quinolyl, isoquinolyl, pyrrolinyl, pyrrolidinyl, dihydro- and tetrahydrofuranyl, dihydro- and tetrahydrothienyl, pyranyl, piperidinyl, morpholinyl, pyrazolinyl, thiazolinyl, oxazolinyl, triazolinyl, and ribofuranosyl. The rings defined by $R_1$, when substituted, are preferably substituted with lower alkyl, lower alkoxy, halogen, hydroxy, amino, carbethoxy, substituted carbamoyl, or hydroxy-lower alkyl, more preferably halogen, phenyl, halophenyl, methylaminophenyl, furanyl, and β-D-ribofuranosyl. Of the halogens, fluorine, chlorine and bromine are especially useful, with fluorine being the most preferred.

A has a sufficient number of chain atoms to complete, together with the two carbon atoms of the triazolopyrimidine ring to which it is attached, a cyclohexene, cyclopentene, piperideine (especially $\Delta^3$-piperideine), tetrahydrobenzo[b]thiophene (especially 1,2,3,4-tetrahydrobenzo[b]thiophene), pyridine, cycloheptene, dihydropyrrole (especially 2,5-dihydropyrrole), isoxazole, oxazole, isothiazole, thiazole, pyrazole, oxathiazole, dithiazole, pyrrole, furan, thiophene, oxazine, thiazine, pyridazine, pyran, thiopyran, oxathiin, dioxin, dithiin, pyrazine, pyrimidine or imidazole group and benzocondensated and/or partially saturated benzocondensated derivatives. The aroyl substituent on A is benzoyl.

Especially preferred are the compounds of formula Ia wherein (1) A is $-(CH_2)_5-$, X is oxygen, $R_1$ is 2-fluorophenyl and (2) A is $-(CH_2)_4-$, X is NH, $R_1$ is furan-2-yl. Other important compounds include those of formula Ia wherein X is oxygen, $R_1$ is 2-fluorophenyl and A is either $-(CH_2)_4-$ or has sufficient chain atoms to complete a 1,2,5,6-tetrahydropyridine ring which is N-substituted with benzyl. Still other important compounds are set forth in the Examples.

The claimed compounds may form acid addition salts, preferably pharmaceutically acceptable acid addition salts. The acid addition salts may be formed by the addition of inorganic or organic acids.

Examples of inorganic salts include halide salts, especially chlorides and sulfates. The organic acids may contain, for example, lower alkyl or aryl groups to which are attached one or more carboxy or sulfo groups. Some examples of organic salts include, for example, the acetate, methanesulfonate, toluenesulfonate, fumarate, cinnamate and benzoate salts.

The compounds of the formulae Ia-Id and their pharmaceutically acceptable salts, and especially those wherein X represents O are benzodiazepine agonists/antagonists and anxiomodulating agents. In addition,

these compounds, and especially those wherein X represents the imino or a substituted imino group, are adenosine antagonists, anti-asthmatic agents, and central nervous stimulating agents ; they enhance cognitive ability. These utilities manifest themselves in mammals such as human beings when administered orally, intraperitoneally or by inhalation in doses of 0.01 mg/kg to 500 mg/kg body weight, preferably 0.1 to 100 mg/kg, and most preferably 10 to 30 mg/kg.

The compounds of the invention bind to the benzodiazepine receptors and exhibit e.g. anxiolytic and/or anticonvulsant effects, or antagonism of the effects of benzodiazepine drugs. Said effects are demonstrable by in vitro and in vivo tests, using advantageously mammals, e.g. mice, rats or monkeys, as test objects. Said compounds can be applied to them enterally or parenterally, advantageously orally, or subcutaneously, intravenously or intraperitioneally, for example, within gelating capsules or in the form of aqueous solutions or suspensions respectively. The applied dosage may range between about 0.1 and 100 mg/kg/day, preferably between about 0.5 and 50 mg/kg/day, advantageously between about 1 and 25 mg/kg/day. The applied dosage in vitro may range between $10^{-5}$ and $10^{-10}$ M concentration, preferably between about $10^{-7}$ and $10^{-9}$ M.

The benzodiazepine receptor binding properties indicative of the nervous system regulatory activity of said new compounds are determined in the receptor binding assay in vitro, e.g. similarly to that in Nature 266, 732 (1977) or Proc. Nat. Sci. USA 74, 3805 (1977). When tritiated flunitrazepam is used, the interaction of other drugs with said receptor can be readily assessed thus : Synaptosomal membranes from rat forebrain are incubated at 0-5° for 30 minutes with 0.5 nM tritiated flunitrazepam and various concentrations of the test substance in a buffer medium maintained at pH 7.5. Solutions of the various concentrations of the test substance are prepared by dilution of a 4.2 mM stock solution in dimethylacetamide-ethanol (1 : 10) with 50 mM pH 7.5 Tris-HC1 buffer. The membranes, containing the receptors with various amounts of tritiated flunitrazepam, are filtered onto glass fiber filters, which are then analyzed in a liquid scintillation counter. The concentration of the compounds of this invention, required to inhibit the specific binding of 0.5 nM of tritiated flunitrazepam by 50%, i.e. the $IC_{50}$, is determined graphically.

In vivo benzodiazepine receptor binding is determined essentially as described in Eur. J. Pharmacol. 48, 213 (1978) and Nature 275, 551 (1978). Test compounds in a corn starch vehicle are administered orally or intraperitoneally to mice or rats. Thirty minutes later, $^{3}H$-flunitrazepam (2 nmoles/kg in saline) is injected into the tail vein, and the animals are sacrificed 20 minutes after injection of flunitrazepam. The brains are then assayed by determining radioactivity in a liquid scintillation counter for binding of the radioligand to the receptors. A decrease in the binding of $^{3}H$-flunitrazepam in the drug-treated animals (as compared with the binding observed in animals treated with vehicle alone) is indicative of benzodiazepine receptor binding by the test compound.

Anxiolytic effects are observed, for example, according to the Cook-Davidson conflict procedure, using male Wistar rats which are maintained at 80% of normal body weight by dietary-, but not water restriction. They are trained to press a lever within a conditioning chamber, also containing a liquid dipper, a house light, a speaker and a grid-floor. Both lever and grid are connected to an electrical shock source and the chamber is situated in a sound-attenuated room in which a white noise-source is activated during testing, in order to mask any extraneous auditory cues. Each session of 47 minutes duration consists of two alternating schedules. The first is a Variable Interval (VI) schedule of 30 seconds, lasting for 5 minutes, during which a sweetened, condensed milk reinforcement is delivered following the first lever-press after average of 30 seconds have elapsed, and a drug induced decrement of this performance is taken as an indication of a neurological deficit. Immediately following the VI-schedule both a 1000 Hz tone and a light-cue are activated, indicating the commencement of the second Fixed Ratio (FR) schedule, lasting for 2 minutes, wherein the milk reinforcement is delivered concomitant with an electric foot shock immediately following the tenth response, thereby establishing a conflict situation. The intensity of said shock ranges between 2.0 and 3.6 mA, varying with each animal, in order to adjust them to about 25-100 responses during this schedule over the entire session. A drug-induced enhancement of performance during the FR-schedule is taken as indication of antianxiety effects. This increased performance is measured by the increased number of electric foot shocks taken during six FR sessions lasting 2 minutes each.

Anticonvulsant effects are observed, for example in the standard Metrazole (pentylenetetrazole) and maximal electroschock tests for assessing anticonvulsant activity, e.g. orally in the rat.

Male Wistar rats (130-175 g) are fasted for 18 hours but allowed water as desired prior to testing. The test compound is administered in a cornstarch vehicle by oral intubation in a volume of 10 ml/Kg of body weight. One hour after administration of the test compound the animals are administered intravenously (caudal vein) a dose of 24 mg/kg of Metrazole in water in a volume of 2.5 ml/kg of body weight. The rats are immediately placed in plexiglass cylinders and observed for clonic seizures of at least 5 seconds duration during the next 60 seconds. The $ED_{50}$ is the dose at which half the animals are protected from Metrazole

induced clonic seizures during the observation periods.

Benzodiazepine antagonism is measured by the antagonism of the anticonvulsant activity of diazepam in the rat Metrazole model. Diazepam (5.4 mg/kg/po) and test compound are administered 1 hour before the Metrazole challenge.

In the maximal electroschock procedure for assessing anticonvulsant activity in rats, seizures are induced by applying 150 mA of electric current for 0.2 seconds through corneal electrodes two hours after oral administration of test compound as described for the Metrazole test above. The $ED_{50}$ is the dose at which half the animals are protected from electroschock induced seizures during the 5 second observation period.

The pharmacological benzodiazepine agonist and/or antagonist profile of the compounds of the invention can also be determined by measuring their effects on a rat brain membrane preparation on the displacement of $^{3}$H-flunitrazepam in the presence or absence of gamma-aminobutyric acid (GABA), on the enhancement of $^{3}$H-muscimol binding by etazolate, or on the binding by etazolate, or on the binding of $^{35}$S-Butyl bicyclophosphorothionate (TBPS).

Accordingly, the compounds of the invention are useful nervous system active agents, e.g. as benzodiazepine receptor agonist/antagonists for example in the treatment or management of nervous system disorders, such as anxiety, convulsive conditions (epilepsy) or other disorders in mammals responsive to said benzodiazepine agonists/antagonists. They are also useful in the preparation of other valuable products, especially of pharmacologically active pharmaceutical compositions.

These and other methods are detailed more fully in Woods, J. Pharmacology and Experimental Therapeutics, Vol. 231, No. 3, 572-576 (1984).

Compounds which are benzodiazepine agonists include those of formula Ia wherein X is oxygen, $R_2$ is hydrogen, $R_1$ is 2-furanyl or optionally substituted phenyl and A completes a cycloheptene ring or an optionally substituted, preferably N-benzyl substituted, 1,2,5,6-tetrahydropyridine ring. Specific benzodiazepine agonists include the compounds of Example 8, 10, 11, 12 and 25.

A mixed benzodiazepine agonists/antagonist of the invention is 2-(2-fluorphenyl)-7,8,9,10-tetrahydro-[1,2,4]triazolo[1,5-c]quinazoline-5(6-H)one, set forth in Example 2.

The compounds of the invention, especially when X is imino or substituted imino, act as adenosine antagonists. Adenosine antagonism is assessed by determination of inhibition of adenosine activation of adenylate cyclase in vesicular preparations from guinea pig brains essentially as described in J. Neurochem. 22, 11031 (1974) and J. Neurochem. 38, 1437 (1982).

A specific adenosine antagonist of the invention is 5-amino-2-(2-furyl)-7,8,9,10-tetrahydro[1,2,4]triazolo[1,5-c]quinazolinemethanesulfonate.

The invention relates particularly to compounds of the formulae Ia-Id, wherein X represents O, S, NH or NR ; R is lower alkyl, aryl-lower alkyl, amino-lower alkyl, or lower alkylamino-lower alkyl, $R_1$ represents phenyl or phenyl substituted by one to three groups selected from lower alkyl, lower alkoxy, hydroxy, halogeno and trifluoromethyl ; or $R_1$ represents a heterocyclic radical bonded by way of a carbon atom, said heterocyclic radical being a five or six membered ring which is aromatic or saturated either partially or totally; the heterocyclic group being unsubstituted or substituted by hydroxy, lower alkyl or halogen ; and wherein $R_2$ represents hydrogen, lower alkyl, aryl-lower alkyl, lower alkenyl, aryl-lower alkenyl, or aryl ; A is a bivalent bridging group of atoms selected from C, N, O, and S which form together with the two adjacent carbon atoms to which they are attached an optionally substituted, mono- or bicyclic aliphatic carbocyclic, or a heterocyclic ring or an optionally substituted mono- or bicyclic heteroatomic ring, the optional substituents of which are selected from lower alkyl, lower alkoxy, hydroxy, halogen, halo (preferably fluoro)-lower alkyl, lower alkylthio, lower alkylsulfonyl, lower alkylsulfinyl, aryl-lower alkyl, amido, carbonyl, carbo-lower alkoxy, and aroyl ; and pharmaceutically acceptable salts thereof.

The invention more particularly relates to compounds of the formulae Ia-Id, wherein X represents O, especially when $R_2$ represents phenyl or substituted phenyl, NH or NR wherein R represents lower alkyl, especially when $R_1$ represents an aromatic heterocyclic group or a substituted aromatic heterocyclic group, $R_1$ represents phenyl or phenyl substituted by one to three groups selected from lower alkyl, for example methyl or ethyl ; lower alkoxy, for example methoxy ; hydroxy ; halogeno, for example fluoro or chloro ; and trifluoromethyl ; or a 5- or 6- membered aromatic heterocyclic radical bonded by way of a ring carbon atom; for example 2- or 3-thienyl ; 2- or 3-furyl ; 2- or 3-pyrrolyl ; 2-, 3- or 4-pyridyl ; 3- or 4-pyrazolyl, or 2- or 4(imidazolyl) ; said aromatic heterocyclic radical being unsubstituted or substituted by hydroxy ; by lower alkyl, for example methyl or ethyl ; or by halogeno, for example fluoro or by chloro, $R_2$ represents hydrogen or lower alkyl, A has a sufficient number of chain atoms to complete, together with the two carbon atoms of the triazolopyrimidine, a cyclohexane, cyclopentene, piperideine (especially $\Delta^3$-piperideine), tetrahydrobenzo[b]thiophene (especially 1,2,3,4-tetrahydrobenzo[b]thiophene), pyridine, cycloheptene, dihydropyrrole (especially 2,5-dihydropyrrole), isoxazole, oxazole, isothiazole, thiazole, pyrazole, oxthiazole, dithiazole, pyrrole, furan, thiophene, oxazine, thiazine, pyridazine, pyran, thiopyrane, oxathiin, dioxin, dithiin, pyrazine, pyrimidine or imidazole group and benzocondensated and/or partially saturated benzocondensated derivatives, and the optional substituents of which are selected from lower alkyl, lower alkoxy, halo-lower alkyl, aryl-lower alkyl or carbo-lower alkoxy, and pharmaceutically acceptable salts thereof.

The invention relates most particularly to compounds of formulae Ia-Id wherein $R_1$ represents phenyl or phenyl substituted by halogeno especially in the ortho or meta positions, particularly fluoro or chloro ; or furyl, especially 2-furyl ; $R_2$ represents hydrogen ; X represents oxygen, especially when $R_1$ represents phenyl substituted by halogeno, for example ortho or meta fluoro ; or X represents NH, especially when $R_1$ is 2-furyl ; and A is a bivalent bridging group of atoms selected from C, N, O, and S which form together with the two adjacent carbon atoms to which they are attached a cyclohexene, cyclopentene, piperideine (especially $\Delta^3$-piperideine), tetrahydrobenzo[b]thiophene (especially 1,2,3,4-tetrahydrobenzo[b]thiophene), pyridine, cycloheptene, dihydropyrrole (especially 2,5-dihydropyrrole), pyrazine, pyrimidine or imidazole group, and the optional substituents of which are selected from lower alkyl, lower alkoxy, halo-lower alkyl, aryl-lower alkyl or carbo-lower alkoxy, and pharmaceutically acceptable salts thereof.

The invention is especially related to the specific compounds mentioned in the examples.

The compounds of the present invention can be prepared by methods known in the prior art. In addition, compounds having formula Ia-Id wherein X=O or S may be prepared by the following methods. The structures shown here will result in compounds of formula Ia. The corresponding products of forumulas Ib-Id can be obtained by utilizing the corresponding starting materials.

The process may consist of variations of the following steps :

a) cyclizing a compound of the formula :

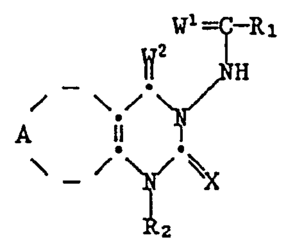

(II),

wherein $R_1$, $R_2$, X and ring A are as defined above, one of $W^1$ and $W^2$ is NH and the other one of $W^1$ and $W^2$ represents O or NH, by treatment with a base furnishing a nitrogen atom, or

b) cyclizing a compound of the formula :

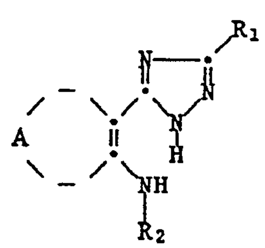

(III),

wherein $R_1$, $R_2$ and A are as defined above, by treatment with a reactive derivative of carbonic acid, or

c) to obtain a compound wherein $R_2$ is hydrogen, reacting a compound of the formula :

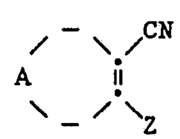

(IV)

wherein A is as defined above and Z is the radical of a carbonic acid derivative bonded via a nitrogen atom, with a hydrazide of the formula :

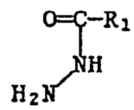

(V)

wherein $R_1$ is as defined above, or

d) to obtain a compound of formula I wherein $R_2$ represents hydrogen and X is oxygen, treating a compound of the formula :

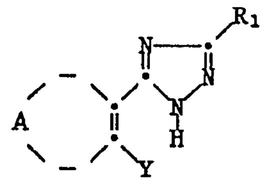

(VI),

wherein Y represents a group capable of being converted to the grouping –N=C=O by an oxidizing agent, with said oxidizing agent followed by ring closure, or

e) to obtain a compound of formula 1, wherein $R_2$ is hydrogen and X is NH, converting a compound of the formula :

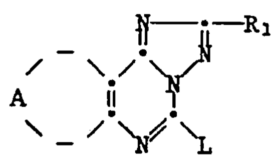

(VII),

Wherein L is selected from halogen, $C_1$-$C_7$-alkoxy, phenyl-$C_1$-$C_7$-alkoxy, mercapto, $C_1$-$C_7$-alkylthio and isothiocyanato, by replacing the group L by the grouping NH, or

f) to obtain a compound of formula I wherein $R_2$ represents hydrogen, treating a compound of the formula :

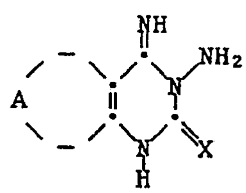

(VIII)

with a reactive derivative of a carboxylic,acid of the formula $R_1$–COOH, and if desired, converting a resulting compound into another compound as defined above and/or converting a resulting salt into the free compound or into a different salt and/or converting a resulting free compound having a salt forming group into a salt.

Process a) : The base furnishing a nitrogen atom is e.g. an amine, preferably a tertiary amine, e.g. pyridine or triethylamine, but may also be ammonia.

The source of ammonia may be, for example, a carbamate, such as a lower alkyl carbamate, for example methyl- or ethylcarbamate or ammonium carbonate. Reactions may be run with or without an inert solvent at atmospheric pressure or elevated pressure, for example in a sealed tube.

Compounds of formula II may be prepared by treating a compound of formula

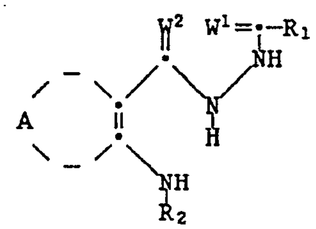

(IX),

wherein $W^1$ and $W^2$ independently represent O or NH with ammonia and/or with a reactive derivative of carbonic acid.

The reactive derivatives of carbonic acid include esters, amides and anhydrides of carbonic acid as well as the corresponding thio- or imino-compounds, such as, for example, phosgene, diethylcarbonate, thiophosgene, trichloromethyl chloroformate, ethyl carbamate, urea, cyanamide or guanidine.

The source of ammonia may be the same as the reactive derivative of carbonic acid, for example a lower alkyl carbamate such as ethyl carbamate.

When $W^1$ and $W^2$ represent O, compounds of the formula IX are prepared from an anthranilic ester and a hydrazide of the formula V ($R_1CONHNH_2$). When $W^1$ and $W^2$ represent NH, compounds of the formula IX are prepared from an anthranilonitrile and a hydrazidine of the formula

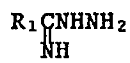

or a hydrazidine of anthranilic acid and a nitrile of the formula $R_1CN$. When $W^1$ represents NH and $W^2$ represents 0, compounds of the formula IX are prepared from an anthranilic hydrazide and a nitrile of the formula $R_1CN$. When $W^1$ represents O and $W^2$ represents NH, IX is prepared from a hydrazidine of an anthranilic acid and a reactive derivative of the acid of the formula $R_1COOH$, e.g. a halide thereof, such as $R_1COCl$ or $R_1COBr$ or an anhydride of the formula $(R_1CO)_2O$.

Process b) : The reactive derivatives of carbonic acid and the general reaction conditions are those described under process a), a further reactive derivative may be a cyanogen halide, e.g. cyanogen chloride or preferably cyanogen bromide. A suitable base may be added to neutralize the hydrohalide formed during the reaction such as triethylamine, pyridine or sodium hydroxide. The cyclization preferably occurs in situ and may be catalyzed by an acid, such as mineral acids, e.g. hydrochloric acid, or a base such as a trialkylamine, e.g. triethylamine.

The compounds of formula III may be produced according to the method of Potts et al, J. Org. Chem. 35, 3448 (1970). They may also be prepared by treating a reactive precursor of an anthranilic acid, such as an isatoic anhydride, with a hydrazidine of the formula

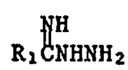

or by reacting a thioamide of the formula

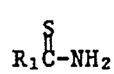

with an anthranilic acid hydrazide

Process c) : The radical of a carbonic acid Z may be selected from isocyanato or isothiocyanato ; –NHC(=O)O-lower alkyl or –NHC(=S)O-lower alkyl ; or –NHC(=O)N-di-lower-alkyl or NHC(=S)N-di-lower-alkyl or corresponding imido derivatives such as cyanimido (–NH–CN).

The reaction takes place preferably in an inert solvent, such as an ether solvent, for example dioxane or an alcohol solvent, for example 2-methoxyethanol, or a liquid amide, for example dimethylacetamide.

When Z represents isocyanato or isothiocyanato ; or –NHC(=O)O-lower alkyl or –NHC(=S)O-lower alkyl, the reaction may be performed in the presence of a base such as a tertiary amine, for example trimethylamine, triethylamine, and, especially, tripropylamine. The compounds of the formula IV wherein Z

represents isocyanato and isothiocyanato may be converted into the corresponding compounds wherein Z represents –NHC(=O)O-lower alkyl and –NHC(=S)O-lower alkyl, respectively, by treatment with a lower alkanol such as ethanol.

The compounds of the formula IV wherein Z represents –NHC(=O)O-lower alkyl or –NHC(=S)O-lower alkyl may also be formed by treating an o-aminobenzonitrile with lower alkyl chloroformate or thiochloroformate, for example ethyl chloroformate or ethyl thiochloroformate.

The compounds of the formula IV wherein Z represents NHC(=O)N-di-lower-alkyl or NHC(=S)N-di-lower alkyl may be formed by treating the appropriate o-isocyanatobenzonitrile or o-isothiocyanatobenzonitrile with a di-lower-alkylamine such as diethylamine.

The preferred solvents when Z represents isocyanato or isothiocyanato are ether solvents, especially dioxane and mostly preferred amides, e.g. 1-methyl-2-pyrrolidinone. The preferred solvents when Z represents –NHC(=O)O-lower alkyl or –NHC(=S)O-lower alkyl or NHC(=O)N-di-lower alkyl or NHC(=S)N-di-lower alkyl are alcohols especially 2-methoxyethanol and mostly preferred amides, e.g. 1-methyl-2-pyrrolidinone or dimethylacetamide. The reaction is preferably conducted at temperatures of 0 to 250°C, preferably 20 to 150°C.

For compounds wherein X denotes NH the starting materials needed are e.g. o-cyanimidobenzonitriles. They are described by Wentrup in Tetrahedron 27, 367 (1971) and by Bedford et al. in J. Chem. Soc., 1633 (1959).

A believed intermediate step of said cyclisation and integrated part of this process is the double cyclisation of a compound of the formula X

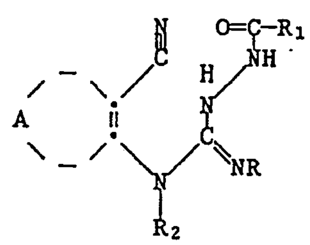

(X)

wherein $R_1$, $R_2$, R and ring A are as defined above.

Process d) : A group capable of being converted to the grouping –N=C=O by an oxidizing agent may be any group that is of significance for the rearrangement reactions such as Hofmann, Curtius or Lossen rearrangement, being e.g. carbamoyl, N-hydroxycarbamoyl or azidocarbonyl.

The oxidizing agent may be, for example, lead tetraacetate or a hypohalite. The hypohalite is preferably an alkali metal hypohalite such as sodium hypochlorite or sodium hypobromite. The function is believed to undergo e.g. the first stage of the Hofmann reaction (Ber. 14, 2725 (1881)), forming the isocyanate which then reacts with the free NH of the triazole.

Process e) : A compound of formula VII, wherein L denotes halogen, lower alkoxy or aryl-lower alkoxy may be hydrolyzed. The hydrolysis is preferably effected by base, for example with aqueous sodium hydroxide.

The 5-halo compounds may be prepared by reacting a compound of formula I wherein X represents O with a reactive halide such as phosphoryl chloride, phenylphosphonic dichloride or phosphorous pentachloride with or without an inert solvent.

The 5-lower alkoxy or 5-aryl-alkoxy compounds may be prepared from the 5-halo compounds by treatment with the appropriate alcohol in the presence of base.

Furthermore, a 5-halo, 5-mercapto or 5-alkylmercapto group in an e-fused [1,2,4]triazolo[1,5-c]pyrimidine is displaced by ammonia or substituted ammonia to form compounds of formula I wherein X represents NR. The mercapto group may be substituted by lower alkyl, for example a methyl group, by reaction of the 5-mercapto compound with e.g. methyl chloride in the presence of a base such as sodium hydride.

The compound of formula VII wherein L represents -SCN may be treated with ammonia or an amine $H_2NR$, in a polar, aprotic solvent, preferably at or near room temperature. The 5-SCN compounds may be prepared from the corresponding 5-thione by treatement of the 5-thione with cyanogen bromide in the presence of a base such as, for example, sodium hydride.

Process f) : A reactive derivative of a carboxylic acid of the formula $R_1$–COOH may be selected from the corresponding acid halides, e.g. acid chlorides, acid esters, such as lower alkyl esters, iminoethers, iminothioethers, thioamides and, preferably, amidines.

The reaction may be performed in an inert solvent, such as an amide, e.g. dimethylacetamide, preferably under heating.

The starting materials of formula VIII may be prepared by reacting a compound of formula IV, wherein ring A is as defined above and Z is selected from the groupings –N=C=S, –N=C=O, –NC-C≡N and –N=C=N-R, with hydrazine or a reactive derivative thereof, e.g. at room temperature in an inert solvent, such as an ether, e.g. tetrahydrofuran. Said process is preferred for manufacture of compounds of formula I wherein X represents oxygen and, especially, sulfur.

Furthermore, one resulting compound of formula I may be converted into another compound of the invention. For example, the 5-thiono compounds may be converted into the 5-oxo compounds by treatment with a hypohalite salt such as sodium hypochlorite or sodium hypobromite. The 5-imino or substituted imino compounds may be hydrolyzed to the corresponding 5-oxo compound with aqueous acid. Compounds I wherein $R_2$ represents hydrogen and X represents oxygen may be converted to compounds wherein $R^2$ represents lower alkyl, for example by reaction with an alkyl halide in the presence of base, such as sodium alkoxide, in an inert solvent, such as dimethyl sulfoxide.

In the above-described preparations of the compounds of the invention, the reactions are conducted under standard conditions. For example, the reaction mixtures may be cooled or heated to appropriate temperatures, appropriate solvents and catalysts may be added, and the reaction may be conducted under an inert atmosphere.

The claimed salts and claimed neutral compounds are interconvertable. For example, acid addition salts may be converted into neutral compounds by treatment with an appropriate base, and neutral compounds may be converted into the corresponding acid addition salt by treatment with the corresponding acid.

The starting marerials for the preparations of the compounds of the invention are either known or may be prepared by methods known in the art.

The compounds of formula Ia-Id are formulated into pharmaceutical compositions comprising an effective amount of the triazolopyrimidine compounds of formula Ia-Id or a salt thereof in combination with conventional excipients or carriers suitable for either enteral or parenteral, such as oral, bronchial, rectal or intravenous, administration. Preferred are tablets, dragées and gelatine capsules comprising the active ingredient together with a) diluents, e.g. lactose, dextrose, sucrose, mannitol, sorbitol, cellulose, calcium phosphates and/or glycine, b) lubricants, e.g. silica, talcum, stearic acid, its magnesium or calcium salt and/or polyethyleneglycol ; for tablets also, c) binders, e.g., magnesium aluminum silicate, starch paste, gelatin, tragacanth, methylcellulose, sodium carboxymethylcellulose and/or polyvinylpyrrolidone ; if desired, d) disintegrants, e.g. starches, agar, alginic acid or its sodium salt, or effervescent mixtures and/or, e) absorbents colorants, flavors and sweeteners. Dragée or tablet cores may be provided with suitable coatings, which may be resistant to gastric juices. Coating solutions are, for example concentrated aqueous sugar solutions, which may contain gum arabic, polyvinylpyrrolidone, polyethylene glycol, talcum and/or titanum dioxide. Resistant coatings are obtained with lacquer solutions in organic solvents, such as shellac, acetylcelullose phthalate or hydroxypropylmethyl cellulose phthalate in ethanol. Dyestuffs or pigments may be added for identification of brand name and dose. Capsules are either made from hard gelatine, or they are soft, closed capsules made from gelatin and a softener, e.g., glycerin or sorbitol. The hard capsules contain either umcompressed powder mixtures, e.g. those mentioned under a) and b), or granulates similar to those used for tablets. In the soft capsules said active ingredients are preferably dissolved or suspended in suitable liquids, such as fatty oils, paraffins or polyethylene glycols. Suppositories are advantageously solid, fatty emulsions or suspensions, containing the active ingredient, for example, in natural or synthetic triglycerides, paraffins, waxes and/or polyethylene glycols.

Compositions for parenteral administration are preferably aqueous solutions or suspensions of said active substances, but also oily solutions or suspensions thereof, e.g., in nautral or synthetic fatty oils, such as sesame oil or ethyl oleate, in suitable ampoules.

Bronchial compositions are preferably aerosol sprays and may be administered from dispenser such as is described in U.S. Patents 4,292,966, 4,174,712, and 4,137,914. The active ingredient is mixed with a propellant such as a hydrocarbon, chlorofluorocarbon mixture, or carbon dioxide.

Said compositions may be sterilized and/or contain adjuvants, such as preserving, stabilizing, wetting or emulsifying agents, solution promoters, salts for regulating the osmotic pressure and/or buffers. They may also contain other valuable substances, and are prepared according to convetional mixing, granulating or coating methods respectively. They may contain from about 10 to 95%, preferably from about 20 to 70% of the active ingredient. Individual unit dosages thereof for a mammal of about 50-70 kg weight may contain preferably between about 10 and 200 mg., advantageously about 20 to 100 mg of said active ingredients.

Having generally described the invention, a more complete understanding can be obtained by reference to certain specific examples, which are provided herein for purposes of illustration. Within the Examples,

numbering of the ring systems is in accordance with the generally accepted rules of nomenclature. The temperatures are given in degrees celsius.

Examples

Example 1: A mixture of 3-(5-aminoimidazol-4-yl)-5-phenyl-1,2,4-triazole (8.5 g), trichloromethylchloroformate (7.0 g) and dioxane (400 ml) is stirred under nitrogen at 20° for 64 hours. To this is added triethylamine (5.15 ml) and the mixture stirred at 20° for 4 hours. The solid is collected, washed with water and air dried. The product is recrystallized from a mixture of dimethylformamide-ether, digested with warm methanol, filtered, washed with ether and oven dried at reduced pressure to afford 2-phenyl-[1,2,4]triazolo-[5,1-i]purin-5(6H)one as a hemihydrate, mp above 300°.

The starting material is prepared in the following way:

A mixture of 6-hydrazinopurine, prepared as described by Montgomery and Holum, J. Amer. Chem. Soc. 79, 2187 (1957), (13.5 g) diphenylether (300 ml), 1 molar p-toluenesulphonic acid in isopropanol (10 ml) and trimethylorthobenzoate (81 ml) is stirred over 2.5 hours at 180° in a apparatus containing a solvent removal trap. The mixture is cooled, diethyl ether (400 ml) added and the resulting solid stirred in hot ethanol (200 ml) for several minutes and collected. This product (12.1 g) is heated with 2.5 molar HCl in isopropanol for 18 hours at 60°, cooled, collected, washed with a little cold water and stirred 5 minutes with 10 ml of 5% aqueous sodium carbonate solution to afford the above triazole, mp 278-280°. The hydrochloride of this product melts in the range from 324 to 326°.

Example 2: The ethyl carbamate of 3,4,5,6-tetrahydroanthranilonitrile (10 g), o-fluorobenzhydrazide (7.94 g), tri-n-propylamine (6.9 ml) and 2-methoxyethanol (170 ml) is stirred at reflux for 18 hours under nitrogen. It is cooled to room temperature and the precipitate which forms is collected, washed with ethanol and dried in vacuo. The product, 2-(2-fluorophenyl)-7,8,9,10-tetrahydro-[1,2,4]triazolo[1,5-c]quinazolin-5(6H)one, melts in the range of 266 to 268°.

The ethyl carbamate is prepared in the following way:

To a solution of sodium (43.5 g) dissolved in absolute ethanol (550 ml) is added 4-amino-3-cyano-$\Delta^3$-piperideine (29.1 g) and diethylcarbonate (280 ml) and the whole stirred at reflux under nitrogen for 2 hours. The solution is ice cooled and glacial acetic acid (145 ml) added cautiously followed by water (600 ml). The aqueous layer is extracted with ether several times and the extract dried over magnesium sulphate and concentrated to dryness at reduced pressure to afford an oil which gradually crystallizes. It is used without further purification (42.2 g).

Example 3: When 2-furoic acid hydroxide is substituted for o-fluorobenzhydrazide in Example 2, 2-(2-furyl)-7,8,9,10-tetrahydro-[1,2,4]-triazolo[1,5-c]quinazolin-5(6H)one is obtained as a white solid, mp 327-330° with decomposition, in 60% yield.

Example 4: A mixture of the ethylcarbamate of 3,4,5,6-tetrahydroanthranilonitrile (8.5 g), o-methylaminobenzhydrazide (7,2 g) and 1-methyl-2-pyrrolidone (80 ml) is stirred under nitrogen at reflux for 16 hours, cooled, diluted with water (300 ml) and the precipitated solid collected, washed with water and pressed dry on the filter. It is washed with ether (100 ml) and oven dried. The free base is suspended in methanol and treated with an equimolar quantity of methanesulphonic acid. The methanesolphonate salt of 2-(2-methylaminophenyl)-7,8,9,10-tetrahydro-[1,2,4]triazolo[1,5-c]quinazolin-5(6H)one crystallizes, mp 257-260°, in 36% yield.

Example 5: When 3-chlorobenzhydrazide is substituted for o-fluorobenzhydrazide in Example 2, 2-(3-chlorophenyl)-7,8,9,10-tetrahydro-[1,2,4]-triazolo[1,5-c]quinazolin-5-(6H)one is obtained and purified by recrystallization from 2-methoxyethanol, mp 342-346° dec. in 50% yield.

Example 6: A mixture of o-fluorobenzhydrazide (8.56 g) 1-amino-2-cyano cyclopenten-1-ylethylcarbamate (10 g, prepared as described by House et al., J.A.C.S. 84, 3139-3147 (1962)), 2-methoxyethanol (185 ml) and tri-n-propylamine (7.4 ml) is stirred under nitrogen at reflux for 20 hours, cooled and treated gradually with water to induce crystallization. It is collected, washed with water, dried and recrystallized from ethanol to afford 8,9-dihydro-2-(2-fluorophenyl)-7H-cyclopenta[e][1,2,4]triazolo[1,5-c]pyrimidin-5(6H)one melting in the range 251 to 253°, in 68% yield.

Example 7: When 2-furoic acid hydrazide is substituted for o-fluorobenzhydrazide in Example 6, 8, 9-dihydro-2-(2-furyl)-7H-cyclopenta-[e][1,2,4]triazolo[1,5-c]pyrimidin-5(6H)one is obtained, mp 297-299°, in 53% yield.

Example 8: A mixture of ethylcarbamate of N-benzyl-3-cyano-4-amino-$\Delta^3$-piperideine (8.2 g), o-fluorobenzhydrazide (84.43 g), 2-methoxyethanol (96 ml) and tri-n-propylamine (3,9 ml) is stirred at reflux under nitrogen for 42 hours. It is cooled and the precipitated solid collected, washed with ethanol, dried and recrystallized from 2-methoxyethanol to afford pure 9-benzyl-2-(2-fluorophenyl)-7,8,9,10-tetrahyd-

ropyrido[3,4-e][1,2,4]triazolo[1,5-c]pyrimidin-5(6H)one, melting in the range 256 to 259°. When treated with an equimolar quantity of methanesulphonic acid in methanol, the free base is converted to the methanesulphonate salt (38%) mp 306-309° after recrystallization from 1 : 1 dimethylacetamide-methanol mixture.

The above ethyl carbamate derivative is prepared by the method described in Example 2 from N-benzyl-3-cyano-4-amino-$\Delta^3$-piperideine (Taylor et al., Tetrahedron 23, 855-890 (1967)) and is obtained as an oil in 94% yield.

Example 9 : When the ethylcarbamate of Example 8 is replaced by N,N'-dicarbethoxy-3-cyano-4-amino-$\Delta^3$-piperideine, 9-carbethoxy-2-(2-fluorophenyl)-7,8,9,10-tetrahydropyrido[3,4-e][1,2,4]-triazolo[1,5-c]pyrimidin-5(6H)one is obtained, mp 307-311° with decomposition, in 46% yield.

The bis-urethane starting material is prepared in the following way :

To a solution of sodium ethoxide in ethanol, prepared from 40.2 g of sodium dissolved in 500 ml of absolute ethanol, is added 3-cyano-4-amino-$\Delta^3$-piperideine (26.9 g, prepared as described by Bachmann and Barker, J. Amer. Chem. Soc. 69, 1535 (1947)) and the whole refluxed 1 hour under nitrogen. Diethylcarbonate (105 ml) is added and the whole stirred at reflux for 2 hours under nitrogen, cooled to room temperature and glacial acetic acid (20 ml) followed by water (1.3 l) is added cautiously. The resulting solution is extracted with ether (4 x 500 ml) and the ether extracts concentrated to about 600 ml, dried over magnesium sulphate and concentrated at reduced pressure to a syrup. Trituration with ether causes precipitation of some solid and concentration of the etheral mother liquor yields a second crop of product, mp 170-172°, suitable for further work. The yield is 68%.

Example 10 : A mixture of the ethylcarbamate of N-benzyl-3-cyano-4-amino-$\Delta^3$-piperideine (5.3 g), benzhydrazide (2.53 g), diemthylacetamide (70 ml) and diisopropylethylamine (0.5 ml) is stirred at reflux under nitrogen for 18 hours. It is concentrated to dryness at reduced pressure, triturated with isopropanol and the resulting solid collected, dried and recrystallized from 2-methoxy-ethanol to afford pure 9-benzyl-2-phenyl-7,8,9,10-tetrahydropyrido[3,4-e]-[1,2,4]triazolo[1,5-c]pyrimidin-5(6H)one, mp 257-259° (72% yield).

Example 11 : A mixture of the ethylcarbamate of N-benzyl-3-cyano-4-amino-$\Delta^3$-piperideine (10 g), p-fluorobenzhydrazide (4.41 g) and 1-methyl-2-pyrrolidine (80 ml) is stirred at reflux under nitrogen for 20 hours. It is evaporated at reduced pressure to remove most of the solvent, then diluted with isopropanol (100 ml) and stirred one-half hour. The precipitated product is collected, washed with isopropanol and dried. It is converted to the p-toluenesulphonate salt by treatment with an equimolar amount of p-toluenesulphonic acid in methanol. The salt is suspended in 2-methoxyethanol, filtered and converted back to the free base in dilute ammonium hydroxide in 42% yield. The pure 9-benzyl-2-(4-fluorophenyl)-7,8,9,10-tetrahydropyrido[3,4-e][1,2,4]triazolo[1,5-c]pyrimidin-5(6H) one melts in the range 252 to 256°.

Example 12 : When 4-chlorobenzhydrazide is substituted for benzhydrazide in Example 10, 9-benzyl-2-(4-chlorophenyl)-7,8,9,10-tetrahydropyrido[3,4-e][1,2,4]triazolo [1,5-c]-pyrimidin-5(6H)one is obtained, melting in the range 250 to 256°, in 54% yield.

Example 13 : When 3-fluorobenzhydrazide is substituted is substituted for p-fluorobenzhydrazide in Example 11, 9-benzyl-2-(3-fluorophenyl)-7,8,9,10 tetrahydropyrido[3,4-e][1,2,4]triazolo[1,5-c]pyrimidine-5(6H)one is obtained and purified as the methanesulphonate salt. After recrystallization from a mixture of dimethylacetamide and methanol, it melts in the range 299 to 302° and is obtained in 30% yield.

Example 14 : A mixture of N,N'-dicarboethoxy-3-cyano-4-amino-$\Delta^3$-piperideine (18.5 g), 2-furoic acid hydrazide (8.73 g), 2-methoxyethanol (220 ml) and tri-n-propylamine (90 ml) is refluxed under nitrogen for 18 hours. It is cooled, concentrated at reduced pressure to a thick slurry, diluted with water (500 ml), stirred for 1 hour and the solid collected, washed with methanol and air dried. The 9-carbethoxy-2-(2-furyl)-7,8,9,10-tetrahydropyrido[3,4-e]-[1,2,4]triazolo[1,5-c]pyrimidin-5(6H)one thus obtained, (11.1 g) decomposes in the range from 316 to 320°.

Example 15 : A suspension of 9-carbethoxy-2-(2-fluorophenyl)-7,8,9,10-tetrahydropyrido[3.4-e][1,2,4]triazolo[1,5-c]pyrimidin-5(6H)one from Example 9 (15.4 g) in 2-methoxyethanol (145 ml) and 4 normal aqueous sodium hydroxide (73 ml) is stirred under nitrogen at 120° for 16 hours. The cooled reaction mixture is gradually brought to pH6 with dilute hydrochloric acid under stirring. The solid material is collected, recrystallized from 2-methoxypropanol and dried in vacuo at 100° for 20° hours. The pure 2-(2-fluorophenyl)-7,8,9,10-tetrahydropyrido[3,4-e][1,2,4]triazolo[1,5-c]-pyrimidin-5(6H)one thus obtained melts in the range from 254-257° (68% yield).

Example 16 : By the method described in Example 15, 2-(2-furyl)-7,8,9,10-tetrahydropyrido[3,4-e][1,2,4]triazolo[1,5-c]pyrimidin-5(6H)one is obtained from the urethane of Example 14, melting in the range 240 to 245° (72% yield).

Example 17 : To a solution of 2-(2-fluorophenyl)-7,8,9,10-tetrahydropyrido[3,4-e][1,2,4]triazolo[1,5-c]pyrimidin-5(6H)one (1.03 g, Example 15), triethylamine (0.9 ml) and 1-methyl-2-pyrrolidinone (18 ml) under nitrogen and magnetic stirring in an ice bath is added benzoyl chloride (0.83 g). It is stirred at room

temperature for 48 hours, then diluted with water and extracted with ethyl acetate. The insoluble material is collected, washed with ether and air dried to afford pure 9-benzoyl-2-(2-fluorophenyl)-7,8,9,10-tetrahydropyrido[3,4-e][1,2,4]triazolo[1,5-c]pyrimidin-5(6H)one, in 83% yield, melting at 342 to 344°.

Example 18 : A mixture of 2-(2-fluorophenyl)-7,8,9,10-tetrahydropyrido[3,4-e][1,2,4]triazolo[1,5-c]pyrimidin-5(6H)one (80.57 g, Example 15) and phenylacetylchloride (3 ml) is heated under nitrogen at 120° for 4 hours. The mixture is diluted with ether (30 ml) and the solid collected, washed with ether and air dried to afford pure 9-phenylacetyl-2-(2-fluorophenyl)-7,8,9,10-tetrahydropyrido[3,4-e][1,2,4]triazolo[1,5-c]pyrimidin-5(6H)one, mp 310-313°, in 85% yield.

Example 19 : A mixture of 2-(2-fluorophenyl)-7,8,9,10-tetrahydropyrido[3,4-e][1,2,4]triazolo[1,5-c]pyrimidin-5(6H)one (0.57 g, Example 15) and phenylisocyanate (3 ml) is heated at 120° for 4 hours, cooled and the resulting solid triturated with ether (30 ml), filtered, washed with ether and dried. The pure 9-phenylureido-2-(2-fluorophenyl)-7,8,9,10-[3,4-e][1,2,4]triazolo[1,5-c]pyrimidin-5(6H)one is thus obtained, mp 291-293°, in 89% yield.

Example 20 : A mixture of ethylcarbamae of N-methyl-3-cyano-4-amino-$\Delta^3$-piperideine (14.6 g), o-fluorobenzhydrazide (10.78 g), tri-n-propylamine (9.5 ml) and 2-methoxyethanol (230 ml) is stirred at reflux under nitrogen for 66 hours, cooled and concentrated to a small volume at reduced pressure. The residue is triturated with isopropanol and the resulting solid collected, washed with methanol and dried. The solid is taken up in methanol, treated with an equimolar quantity of methanesulphonic acid and the resulting salt collected and air dried in vacuo to afford pure 2-(2-fluorophenyl)-9-methyl-7,8,9,10-tetrahydropyrido[3,4-e][1,2,4]triazolo[1,5-c]-pyrimidin-5(6H)one methanesulphonate, mp 285-287°, in 41% yield.

The starting material is prepared by the method described in Example 2 from N-methyl-3-cyano-4-$\Delta^3$-piperideine, prepared as described by Cologne et al., Bull. Soc. Chim. France, 1963, 2264-2270, in 86% yield.

Example 21 : When the ethyl carbamate of N-ethyl-3-cyano-4-amino-$\Delta^3$-piperideine is substituted for the N-methyl compound of Example 20, 9-ethyl-2-(2-fluorophenyl)-7,8,9,10-tetrahydropyrido-[3,4-e][1,2,4]triazolo[1,5,-c]-pyrimidin-5(6H)one methanesulphonate is obtained, mp 184-286°, in 48% yield.

The ethyl carbamate is prepared by the method described in Example 2 from N-ethyl-3-cyano-4-$\Delta^3$-piperideine, prepared as described by Cologne et al., Bull. Soc. Chim. France, 1963, 2264-2270, in 81% yield.

Example 22 : When the ethyl carbamate of N-isopropyl-3-cyano-4-amino-$\Delta^3$-piperideine is substituted for the N-methyl compound of Example 20, 2-(2-fluorophenyl)-9-isopropyl-7,8,9,10-tetrahydropyrido[3,4-e][1,2,4]triazolo[1,5-c]-pyrimidin-5(6H)one methanesulphonate is obtained, mp 300-303°, in 62% yield.

The ethyl carbamate used as starting material is prepared from N-isopropyl-3-cyano-amino-4-$\Delta^3$-piperideine as described in Example 2. This amino compound is prepared from N-isopropyl bis-(β-cyanoethyl) amine as described by Cologne et al., Bull. Soc. Chim. France, 1963, 2264-2270. The amine is prepared by reaction of isopropylamine in water with two moles of acrylonitrile at ambient temperature over 4 days in almost quantitive yield. The material is extracted with ether, the ether solution dried over magnesium sulphate and concentrated to a heavy oil. All intermediates are oils and require no further purification.

Example 23 : When the ethyl carbamate of N-β-phenethyl-3-cyano-4-amino-$\Delta^3$-piperideine is substituted for the N-methyl compound in Example 20, 2-(2-fluorophenyl)-9-β-phenethyl-7,8,9,10-tetrahydropyrido[3,4-e][1,2,4]triazolo[1,5-c]pyrimidin-5(6H)one methanesulphonate is obtained, mp 167-171°, in 38% yield.

The ethyl carbamate starting material was prepared in the same manner as the ethyl carbamate in Example 22, except that β-phenethylamine is substituted for isopropylamine.

Example 24 : When the ethyl carbamate of 1-benzyl-3-amino-4-cyano-2-methyl-$\Delta^3$-pyrroline is substituted for the ethyl carbamate of the N-benzyl compound in Example 11, 8-benzyl-2-(2-fluorophenyl)-7-methyl-7,9-dihydro-8H-pyrrolo[3,4-e][1,2,4]triazolo[1,5-c]-pyrimidin-5(6H)one is obtained, mp 290-293°, after recrystallization from 2-methoxyethanol, in 36% yield.

The ethyl carbamate intermediate is prepared from 3-amino-1-benzyl-4-cyano-2-methyl-$\Delta^3$-pyrroline (Cavalla, J. Chem. Soc. 1962, 4664) as described in Example 2.

Example 25 : A mixture of the ethyl carbamate of 1-amino-2-cyano-cyclohept-1-ene (11.6 g), o-fluorobenzhydrazide (8.74 g) and 1-methyl-2-pyrrolidone (140 ml) is stirred at reflux under nitrogen for 18 hours, cooled and triturated with water to produce solid material. It is recrystallized from (±)1-methoxy-2-propanol to afford pure 2-(2-fluorophenyl)-8,9,10,11-tetrahydro-7H-cyclohepta[e][1,2,4]-triazolo[1,5-c]pyrimidin-5(6H)one, mp 257-259°, in 63% yield.

The starting material ethyl carbamate is prepared from 1-amino-2-cyanocyclohept-1-ene (Krüger, J. Organometal. Chem. 9, 125 (1967) as described in Example 2.

Example 26 : A mixture of ethyl carbamate of 4-amino-3-cyanopyridine (81.3 g), o-fluorobenzhydrazide (1.05 g), 2-methoxyethanol (15 ml) and tri-n-propylamine (0.9 ml) is heated at reflux under nitrogen for 66

hours. One-half of the solvent is removed by evaporation at reduced pressure and the mixture is then cooled, filtered and the precipitate washed with ethanol and dried. After recrystallization from dimethylacetamide-ethanol, it is treated with an equimolar quantity of methanesulphonic acid in methanol and the salt precipitates by addition of ether. The product, (43%) 2-(2-fluorophenyl)-pyrido[3,4-e][1,2,4]triazolo[1,5-c]pyrimidin-5(6H) one methanesulphonate, melts in the range 290 to 293°.

The starting material ethyl carbamate is prepared in the following way : To a heterogeneous mixture of 4-amino-3-cyanopyridine (1.2 g), sodium bicarbonate (1.9 g) and methyl ethyl ketone (30 ml) is added under nitrogen and stirring ethyl chloroformate (1.9 ml). It is refluxed under nitrogen for 20 hours, filtered free of inorganic solid and the filtrate concentrated at reduced pressure. The residual solid is recrystallized from chlorobenzene-cyclohexane to give the desired carbamate, melting in the range 108-120°. This is suitable for the next step.

Example 27 : The methyl carbamate of 2-amino-3-cyano-4,5,6,7-tetrahydrobenzo(b)thiophene (7.08 g), 2-fluorobenzhydrazide (4.66 g), 2-methoxyethanol (100 ml) and tri-n-propylamine (3 ml) are refluxed under nitrogen for 19 hours. The mixture is cooled and the precipitate collected. The mother liquor is treated with methanol (100 ml) and refrigerated 24 hours to produce a second crop of solid. The combined material is recrystallized from 2-methoxyethanol and dried 20 hours at 100°/0.01 mm to afford 2-(2-fluorophenyl)-8,9,10,11-tetrahydro(1)benzothieno[3,4-e][1,2,4]triazolo[1,5-c]pyrimidin-5(6H)one, which decomposes above 316°. The yield is 50%.

The starting material carbamate is prepared in the following way :

To a solution of 2-amino-3-cyano-4,5,6,7-tetrahydrobenzo(b)thiophene (8.9 g, prepared as described by Gewald et al., Chem. Ber. 99, 94 (1966)), in methyl ethyl ketone (150 ml) is added sodium bicarbonate (5 g) followed by methyl chloromate (4.2 ml) and the whole stirred at 80° under nitrogen for 20 hours. It is filtered free of inorganic solid while hot and the filtrate concentrated to dryness at reduced pressure. The residue is recrystallized from chlorobenzene-cyclohexane to afford the pure methyl carbamate, mp 162-164°, in 66% yield.

Example 28 : When the methyl carbamate of 2-amino-3-cyano-4,5-dimethylthiophene is substituted for the carbamate in Example 27, 2-(2-fluorophenyl)-8,9-dimethylthieno[3,4-e][1,2,4]triazolo[1,5-c]-pyrimidin-5(6H)one is obtained, which decomposes above 330°, in 83% yield.

The starting methyl carbamate is obtained from the aminonitrile (prepared by the method of Gewald et al., loc. cit.) as described in Example 27., mp 155-157° after recrystallization.

Example 29 : In a pressure vessel is placed 30 ml of ammonium hydroxide saturated with ammonia at 0° and 5-chloro-2-(2-furyl)-7,8,9,10-tetrahydro[1,2,4]triazolo[1,5-c]quinazoline (0.63 g) and the whole is heated at 150° for 6 hours. The mixture is cooled and the precipitate collected, washed with methanol and recrystallized from 2-methoxyethanol. The free base is converted with methanesulphonic acid in isopropanol to the salt and precipitated with ether. The pure 5-amino-2-(2-furyl)-7,8,9,10-tetrahydro[1,2,4]triazolo[1,5-c]quinazoline methane sulphonate melts in the range 269 to 271°. It is obtained in 38% yield.

The starting chloro compound is prepared as follows :

To a solution of phosphorous pentachloride (0.1 g) in phosphoryl chloride (13.7 ml) is added 2-(2-furyl)-7,8,9,10-tetrahydro[1,2,4] triazolo[1,5-c]quinazolin-5(6H)one (Example 3, 0.64 g), the mixture stirred for 5 minutes and pyridine (0.41 g) added dropwise. It is heated at 110° for 17 hours and then concentrated to dryness at reduced pressure. The residual solid is suspended in ethyl acetate (100 ml) and the mixture washed with cold 2N hydrochloric acid (3 x 30 ml). The organic layer is dried over sodium sulphate and concentrated to dryness at reduced pressure to yield a white solid suitable for the next step, in 93% yield.

Example 30 : A mixture of 3-(5-aminoimidazol-4-yl)-5-phenyl-1,2,4 triazole (11.3 g, from Example 1), methanol (200 ml) and cyanogen bromide (5.25 g) is stirred under nitrogen for 2 hours at 40°. It is cooled and the solid collected, dissolved in 10 N sodium hydroxide solution, filtered and precipitated by addition of glacial acetic acid to pH 5. The solid is washed with water, stirred in a mixture of methanesulphonic acid in methanol for one-half hour and collected (1.39 g), mp > 350°. After drying at room temperature and high vacuum, the hemihydrate of 5-amino-2-phenyl-[1,2,4]triazolo[5,1-i]-purine is thus obtained.

Flunitrazepam binding studies were undertaken with the compounds of Examples 1, 2, 4-9, 13 and 15-30. The results thereof are set forth in the Table below as $IC_{50}$ in nanomolar values.

| Compound of Example # | Flunitrazepam Binding |
|---|---|
| 1 | > 1000 |
| 2 | 1.7 |
| 4 | > 1000 |
| 5 | > 20 |
| 6 | 4.7 |
| 7 | 22.7 |
| 8 | 0.44 |
| 9 | 13.3 |
| 13 | 2.4 |
| 15 | 440 |
| 16 | > 1000 |
| 17 | 50 |
| 18 | 140 |
| 19 | 67 |
| 20 | 180 |
| 21 | 150 |
| 22 | 110 |
| 23 | 28 |
| 24 | > 1000 |
| 25 | 10 |
| 26 | 12 |
| 27 | > 20 |
| 28 | > 20 |
| 29 | 1000 |
| 30 | > 1000 |

Example 31 : A mixture of 2-(2-fluorophenyl)-7,8,9,10-tetrahydro[1,2,4]triazolo[1,5-c]quinazolin-5(6H)one, (Example 2, 1.4 g), and sodium methoxide (400 mg) is dissolved in dry dimethylsulphoxide (80 ml) under nitrogen at 85°, stirred at 50° for 30 min. and then treated dropwise with methyl iodide (0.7 ml) in dimethylsulphoxide (20 ml). It is allowed to stir another hour at 50°, then quenched in ice-water and the resulting white precipitate collected, washed with water and dried at 100° under vacuum. It is recrystallized from 2-methoxyethanol to afford pure 2-(2-fluorophenyl)-6-methyl-7,8,9,10-tetrahydro[1,2,4]triazolo[1,5-c]quinazolin-5(6H)one, mp 236-239°.

Example 32 : To a suspension of 50% sodium hydride in oil (300 mg) in dry dimethylformamide (16 ml) is added 3-(2-amino-pyrazin-3-yl)-5-(2-furyl)-1,2,4-triazole (1.37 g) and the whole stirred under nitrogen at 60° until a solution forms. To this is added cyanogen bromide (700 mg) in dimethylformamide (9 ml) and the whole stirred under nitrogen over 66 hours at 60°. It is cooled to 0° and the solid collected, washed with dimethylformamide, water, then ethanol and finally ether and air dried. It is recrystallized from dimethylacetamide (70 ml) to which methanol (50 ml) is added to form pure 5-amino-2-(2-furyl)-pyrazino[2,3-e][1,2,4]-triazolo[1,5-c]-pyrimidine, melting in the range 347-351°.

The starting material triazole is prepared in the following manner : To a suspension of sodium ethoxide, prepared from sodium (460 mg) in dry ethanol (20 ml) is added 2-furylcarboxamidine hydrochloride (2.9 g) in ethanol (20 ml) under nitrogen and after 5 min. stirring it is filtered and the filtrate added to a mixture of 2-aminopyrazine-3-carbohydrazide (3.1 g), ethanol (10 ml) and chlorobenzene (40 ml). It is heated under nitrogen in an apparatus with a solvent separator at 120° until no more solvent distills, then heated 18 hours at reflux. The mixture is cooled, filtered and the solid washed with methanol (3 × 30 ml) and recrystallized from 2-methoxyethanol to afford the pure triazole, mp 247-250°.

The starting material hydrazide is prepared by reaction of methyl-2-aminopyrazine-3-carboxylate with hydrazine hydrate at 80° for 1 hour. The methyl ester is prepared by reaction of 2-aminopyrazine-3-carboxylic acid with methanol containing concentrated sulphuric acid over 3 days, mp 167-170°.

## Claims

### Claims for the Contracting States BE CH DE FR GB IT LI LU NL SE :

1. A compound of the formula

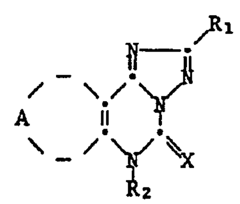

(Ia),

its tautomers and pharmaceutically acceptable salts wherein

X is O, S, NH or NR ;

R is $C_1$-$C_7$-alkyl, $C_2$-$C_7$-alkenyl, $C_2$-$C_7$-alkynyl, wherein the unsaturated bonds of the alkenyl and alkynyl groups are separated from the N atom of X by at least one saturated carbon atom, $C_2$-$C_7$-cycloalkyl or aromatic ring-$C_1$-$C_7$-alkyl wherein the aromatic ring is phenyl, naphthyl or aromatic heteroaryl with 5-7 ring members having at least one heteroatom selected from O, N and S, but not more than one O and S atom, all of the foregoing $C_1$-$C_7$-alkyl groups being optionally interrupted by a heteroatom selected from O, N or S ; or R is hydroxy, hydroxy-$C_2$-$C_4$-alkyl, phenyl, naphthyl or aromatic heteroaryl with 5-7 ring members having at least one heteroatom selected from O, N and S, but not more than one O and S atom, or the group –C(=NH)–$NH_2$ ;

$R_1$ is phenyl, naphthyl, pyridyl, thienyl, furyl, pyrrolyl, thiazolyl, isothiazolyl, oxazolyl, imidazolyl, pyrazolyl, 1,2,3-or 1,2,4-triazolyl, tetrazolyl, pyrimidinyl, quinolyl, isoquinolyl, pyrrolinyl, pyrrolidinyl, dihydro- or tetrahydrofuranyl, dihydro- or tetrahydrothienyl, pyranyl, piperidinyl, morpholinyl, pyrazolinyl, thiazolinyl, oxazolinyl, triazolinyl, or ribofuranosyl, these groups $R_1$ being unsubstituted or substituted by halogen, $C_1$-$C_7$-alkyl, halo-$C_1$-$C_7$-alkyl, hydroxy, $C_1$-$C_7$-alkoxy, hydroxy-$C_1$-$C_7$-alkyl, amino, mono- or di-$C_1$-$C_7$-alkylamino, $C_1$-$C_7$-alkoxycarbonyl, carbamoyl, or $C_1$-$C_7$-alkylcarbamoyl ;

$R_2$ is hydrogen, $C_1$-$C_7$-alkyl, hydroxy-$C_1$-$C_7$-alkyl, $C_2$-$C_7$-alkenyl, aryl-$C_1$-$C_7$-alkyl, aryl-$C_2$-$C_7$-alkenyl or

aryl wherein aryl is phenyl, naphthyl or aromatic heteroaryl with 5-7 ring members having at least one heteroatom selected from O, N and S, but not more than one O or S atom ;

A is a bivalent bridging group containing chain atoms selected from C, O, N and S which forms together with the two adjacent carbon atoms to which the chain is attached a cyclohexene, cyclopentene, piperideine, tetrahydrobenzo[b]thiophene, pyridine, cycloheptene, dihydropyrrole, isoxazole, oxazole, isothiazole, thiazole, pyrazole, oxathiazole, dithiazole, pyrrole, furan, thiophene, oxazine, thiazine, pyridazine, pyran, thiopyran, oxathiin, dioxin, dithiin, pyrazine, pyrimidine or imidazole, a benzocondensated derivative thereof, or a partially saturated benzocondensated derivative thereof, these groups A being unsubstituted or substituted by $C_1$-$C_7$-alkyl, $C_1$-$C_7$-alkoxy, hydroxy, halogen, halo-$C_1$-$C_7$-alkyl, nitro, amino, $C_1$-$C_7$-alkylthio, $C_1$-$C_7$-alkylsulfonyl, $C_1$-$C_7$-alkylsulfinyl, aryl-$C_1$-$C_7$-alkyl wherein aryl is phenyl, naphthyl or aromatic heteroaryl with 5-7 ring members having at least one heteroatom selected from O, N or S, but not more than one O or S atom, amido, carbamoyl, $C_1$-$C_7$-alkoxycarbonyl or benzoyl.

2. A tautomer of a compound of the formula Ia according to claim 1 represented by the formulae :

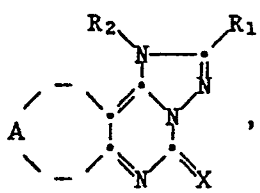
(Ib) ,

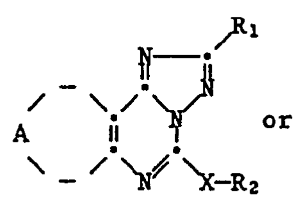
(Ic) or

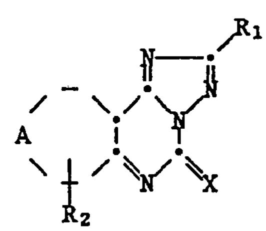
(Id)

3. A compound according to claims I or 2 of the formulae Ia-Id, wherein X represents O, S, NH or NR; R is $C_1$-$C_7$-alkyl, aryl-$C_1$-$C_7$-alkyl wherein aryl is phenyl, naphthyl or aromatic heteroaryl with 5-7 ring members having at least one heteroatom selected from O, N and S, but not more than one O or S atom, amino-$C_1$-$C_7$-alkyl, or $C_1$-$C_7$-alkylamino-$C_1$-$C_7$-alkyl ; $R_1$ represents phenyl or phenyl substituted by one to three groups selected from $C_1$-$C_7$-alkyl, $C_1$-$C_7$-alkoxy, hydroxy, halogen and trifluoromethyl ; or $R_1$ represents a heterocyclic radical bonded by way of a carbon atom, said heterocyclic radical being pyridyl, thienyl, furyl, pyrrolyl, thiazolyl, isothiazolyl, oxazolyl, imidazolyl, pyrazolyl, 1,2,3- or 1,2,4-triazolyl, tetrazolyl, pyrimidinyl, quinolyl, isoquinolyl, pyrrolinyl, pyrrolidinyl, dihydro-or tetrahydrofuranyl, dihydro- or tetrahydrothienyl, pyranyl, piperidinyl, morpholinyl, pyrazolinyl, thiazolinyl, oxazolinyl, triazolinyl, or ribofuranosyl, said heterocyclic group being unsubstituted or substituted by hydroxy, $C_1$-$C_7$-alkyl or halogen ; R2 represents hydrogen, $C_1$-$C_7$-alkyl, aryl-$C_1$-$C_7$-alkyl, $C_2$-$C_7$-alkenyl, aryl-$C_2$-$C_7$-alkenyl or aryl wherein aryl is phenyl, naphthyl or aromatic heteroaryl with 5-7 ring members having at least one heteroatom selected from O, N and S, but not more than one O or S atom ; A is a bivalent bridging group of atoms selected from C, N, O, and S which forms together with the two adjacent carbon atoms to which A is attached a cyclohexene, cyclopentene, piperideine, tetrahydrobenzo[b]thiophene, pyridine, cycloheptene, dihydropyrrole, isoxazole, oxazole, isothiazole, thiazole, pyrazole, oxathiazole, dithiazole, pyrrole, furan, thiophene, oxazine, thiazine, pyridazine, pyran, thiopyran, oxathiin, dioxin, dithiin, pyrazine, pyrimidine or imidazole, a benzocondensated derivative thereof, or a partially saturated benzocondensated derivative thereof, these groups A being unsubstituted or substituted by $C_1$-$C_7$-alkyl, $C_1$-$C_7$-alkoxy, hydroxy, halogen, halo-$C_1$-$C_7$-alkyl, $C_1$-$C_7$-alkylthio, $C_1$-$C_7$-alkylsulfonyl, $C_1$-$C_7$-alkylsulfinyl, aryl-$C_1$-$C_7$-alkyl wherein aryl is phenyl, naphthyl or aromatic heteroaryl with 5-7 ring members having at least one heteroatom selected from O, N and S, but not more than one O or S atom, amido, carbamoyl, $C_1$-$C_7$-alkoxycarbonyl or benzoyl ; and pharmaceutically acceptable salts thereof.

4. A compound according to claims 1 or 2 of the formulae Ia-Id, wherein X represents O, NH or NR wherein R represents C1-$C_7$-alkyl ; $R_1$ represents phenyl or phenyl substituted by one to three groups selected from $C_1$-$C_7$-alkyl, $C_1$-$C_7$-alkoxy, hydroxy, halogen and trifluoromethyl ; or $R_1$ is 2- or 3-thienyl, 2- or 3-furyl, 2- or 3-pyrrolyl, 2-, 3- or 4-pyridyl, 3- or 4-pyrazolyl, or 2- or 4-imidazolyl, said heterocyclic radical being unsubstituted or substituted by hydroxy, $C_1$-$C_7$-alkyl or halogen ; $R_2$ represents hydrogen or $C_1$-$C_7$-alkyl ; and A is a bivalent bridging group of atoms selected from C, N, O and S which forms together with the two adjacent carbon atoms to which A is attached a cyclohexene, cyclopentene, piperideine, tetrahydrobenzo[b-]thiophene, pyridine, cycloheptene, dihydropyrrole, isoxazole, oxazole, isothiazole, thiazole, pyrazole,

oxathiazole, dithiazole, pyrrole, furan, thiophene, oxazine, thiazine, pyridazine, pyran, thiopyran, oxathiin, dioxin, dithiin, pyrazine, pyrimidine or imidazole, a benzocondensated derivative thereof, or a partially saturated benzocondensated derivative thereof, these groups A being unsubstituted or substituted by $C_1$-$C_7$-alkyl, $C_1$-$C_7$-alkoxy, halo-$C_1$-$C_7$-alkyl, aryl-$C_1$-$C_7$-alkyl wherein aryl is phenyl, naphthyl or aromatic heteroaryl with 5-7 ring members having at least one heteroatom selected from O, N and S, but not more than one O or S atom, or $C_1$-$C_7$-alkoxycarbonyl ; and pharmaceutically acceptable salts thereof.

5. A compound according to claims 1 or 2 of the formulae Ia-Id, wherein X represents O or NH ; $R_1$ represents phenyl, phenyl substituted by halo in the ortho or meta position, or 2-furyl ; $R_2$ represents hydrogen; and A is a bivalent bridging group of atoms selected from C, N, O, and S which forms together with the two adjacent carbon atoms to which A is attached a cyclohexene, cyclopentene, piperideine, tetrahydrobenzo[b-]thiophene, pyridine, cycloheptene, dihydropyrrole, pyrazine, pyrimidine or imidazole, these groups A being unsubstituted or substituted by $C_1$-$C_7$-alkyl, $C_1$-$C_7$-alkoxy, halo-$C_1$-$C_7$-alkyl, aryl-$C_1$-$C_7$-alkyl wherein aryl is phenyl, naphthyl or aromatic heteroaryl with 5-7 ring members having at least one heteroatom selected from O, N and S, but not more than one O or S atom, or $C_1$-$C_7$-alkoxycarbonyl ; and pharmaceutically acceptable salts thereof.

6. A compound according to claim 1 of the formula Ia wherein X is oxygen, $R_1$ is fluorophenyl and A is $-(CH_2)_5-$, and pharmaceutically acceptable salts thereof.

7. A compound according to claim 1 of the formula Ia wherein X is NH, $R_1$ is 2-furyl and A is $-(CH_2)_4-$, and pharmaceutically acceptable salts thereof.

8. 9-Benzyl-2-phenyl-7,8,9,10-tetrahydropyrido[3,4-e][1,2,4] triazolo[1,5-c]pyrimidin-5(6H)one and pharmaceutically acceptable salts thereof according to claim 1.

9. 9-Benzyl-2-(3-fluorophenyl)-7,8,9,10-tetrahydropyrido[3,4-e][1,2,4]triazolo[1,5-c]pyrimidine-5(6H)one and pharmaceutically acceptable salts thereof according to claim 1.

10. A pharmaceutical composition containing a compound of the formula Ia, its tautomers or pharmaceutically acceptable salts according to claim 1 together with a pharmaceutically acceptable carrier.

11. A compound according to claims 1-9 for use in the treatment of humans or animals.

12. A compound according to claims 1-9 for use as anxiomodulator.

13. A compound according to claims 1-9 for use as benzodiazepine antagonist.

14. Use of a compound according to claims 1-9 for the preparation of a pharmaceutical composition.

15. A process for the preparation of a pharmaceutical composition according to claim 10, characterized by admixing a compound according to claim 1 with a pharmaceutically acceptable carrier.

16. A process for the preparation of a compound according to claim 1 wherein the groups are as defined in claim 1, which comprises

a) cyclizing a compound of the formula :

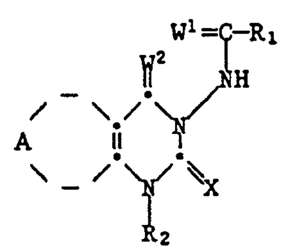

(II),

wherein $R_1$, $R_2$, X and A are as defined above, one of $W^1$ and $W^2$ is NH and the other one of $W^1$ and $W^2$ represents O or NH, by treatment with a base furnishing a nitrogen atom, or

b) cyclizing a compound of the formula :

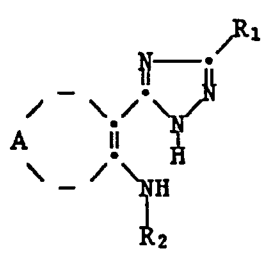

(III),

wherein $R_1$, $R_2$ and A are as defined above, by treatment with a reactive derivative of carbonic acid, or
c) to obtain a compound wherein $R_2$ is hydrogen, reacting a compound of the formula :

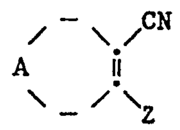

(IV)

wherein A is as defined above and 2 is the radical of a carbonic acid derivative bonded via a nitrogen atom, with a hydrazide of the formula :

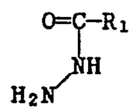

(V)

wherein $R_1$ is as defined above, or
d) to obtain a compound of formula I wherein $R_2$ represents hydrogen and X is oxygen, treating a compound of the formula :

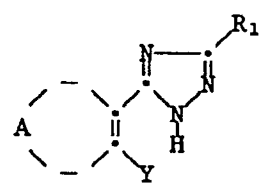

(VI),

wherein Y represents a group capable of being converted to the grouping –N=C=O by an oxidizing agent, with said oxidizing agent followed by ring closure, or
e) to obtain a compound of formula I, wherein $R_2$ is hydrogen and X is NH, converting a compound of the formula :

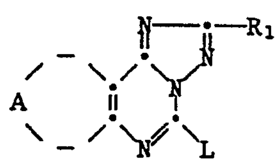

(VII),

wherein L is selected from halogen, $C_1$-$C_7$-alkoxy, phenyl-$C_1$-$C_7$-alkoxy, mercapto, $C_1$-$C_7$-alkylthio and isothiocyanato, by replacing the group L by the grouping NH, or
f) to obtain a compound of formula I wherein $R_2$ represents hydrogen, treating a compound of the formula :

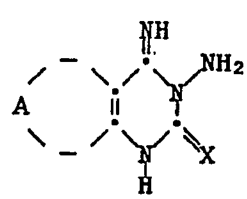

(VIII)

with a reactive derivative of a carboxylic acid of the formula $R_1$–COOH, and if desired, converting a resulting compound into another compound as defined above and/or converting a resulting salt into the free compound or into a different salt and/or converting a resulting free compound having a salt forming group into a salt.

**Claims for the Contracting State AT :**

1. A process for the preparation of a compound of the formula

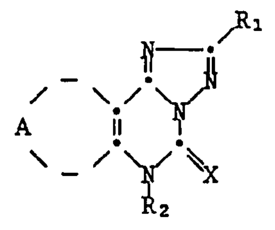

(Ia),

its tautomers and pharmaceutically acceptable salts wherein

X is O, S, NH or NR ;

R is $C_1$-$C_7$-alkyl, $C_2$-$C_7$-alkenyl, $C_2$-$C_7$-alkynyl, wherein the unsaturated bonds of the alkenyl and alkynyl groups are separated from the N atom of X by at least one saturated carbon atom, $C_5$-$C_7$-cycloalkyl or aromatic ring-$C_1$-$C_7$-alkyl wherein the aromatic ring is phenyl, naphthyl or aromatic heteroaryl with 5-7 ring members having at least one heteroatom selected from O, N and S, but not more than one O and S atom, all of the foregoing $C_1$-$C_7$-alkyl groups being optionally interrupted by a heteroatom selected from O, N or S ; or R is hydroxy, hydroxy-, $C_2$-$C_4$-alkyl, phenyl, naphthyl or aromatic heteroaryl with 5-7 ring members having at least one heteroatom selected from O, N and S, but not more than one O or S atom, or the group –C(=NH)–$NH_2$ ;

$R_1$ is phenyl, naphthyl, pyridyl, thienyl, furyl, pyrrolyl, thiazolyl, isothiazolyl, oxazolyl, imidazolyl, pyrazolyl, 1,2,3-or 1,2,4-triazolyl, tetrazolyl, pyrimidinyl, quinolyl, isoquinolyl, pyrrolinyl, pyrrolidinyl, dihydro- or tetrahydrofuranyl, dihydro- or tetrahydrothienyl, pyranyl, piperidinyl, morpholinyl, pyrazolinyl, thiazolinyl, oxazolinyl, triazolinyl, or ribofuranosyl, these groups $R_1$ being unsubstituted or substituted by halogen, $C_1$-$C_7$-alkyl, halo-$C_1$-$C_7$-alkyl, hydroxy, $C_1$-$C_7$-alkoxy, hydroxy-$C_1$-$C_7$-alkyl, amino, mono- or di-$C_1$-$C_7$alkylamino, $C_1$-$C_7$-alkoxycarbonyl, carbamoyl, or $C_1$-$C_7$alkylcarbamoyl ;

$R_2$ is hydrogen, $C_1$-$C_7$-alkyl, hydroxy-$C_1$-$C_7$-alkyl, $C_2$-$C_7$-alkenyl, aryl-$C_1$-$C_7$-alkyl, aryl-$C_2$-$C_7$-alkenyl or aryl wherein aryl is phenyl, naphthyl or aromatic heteroaryl with 5-7 ring members having at least one heteroatom selected from O, N and S, but not more than one O or S atom ;

A is a bivalent bridging group containing chain atoms selected from C, O, N and S which forms together with the two adjacent carbon atoms to which the chain is attached a cyclohexene, cyclopentene, piperideine, tetrahydrobenzo[b]thiophene, pyridine, cycloheptene, dihydropyrrole, isoxazole, oxazole, isothiazole, thiazole, pyrazole, oxathiazole, dithiazole, pyrrole, furan, thiophene, oxazine, thiazine, pyridazine, pyran, thiopyran, oxathiin, dioxin, dithiin, pyrazine, pyrimidine or imidazole, a benzocondensated derivative thereof, or a partially saturated benzocondensated derivative thereof, these groups A being unsubstituted or substituted by $C_1$-$C_7$-alkyl, $C_1$-$C_7$-alkoxy, hydroxy, halogen, halo-$C_1$-$C_7$-alkyl, nitro, amino, $C_1$-$C_7$-alkylthio, $C_1$-$C_7$-alkylsulfonyl, $C_1$-$C_7$-alkylsulfinyl, aryl-$C_1$-$C_7$-alkyl wherein aryl is phenyl, naphthyl or aromatic heteroaryl with 5-7 ring members having at least one heteroatom selected from O, N or S, but not more than one O or S atom, amido, carbamoyl, $C_1$-$C_7$-alkoxy-carbonyl or benzoyl, which comprises

a) cyclizing a compound of the formula :

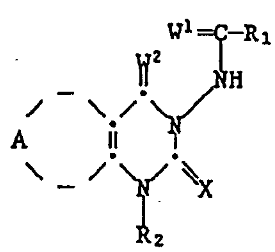

(II),

wherein $R_1$, $R_2$, X and A are as defined above, one of $W^1$ and $W^2$ is NH and the other one of $W^1$ and $W^2$ represents O or NH, by treatment with a base furnishing a nitrogen atom, or

b) cyclizing a compound of the formula :

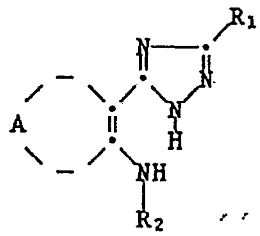

(III),

wherein $R_1$, $R_2$ and A are as defined above, by treatment with a reactive derivative cf carbonic acid, or
c) to obtain a compound wherein $R_2$ is hydrogen, reacting a compound of the formula :

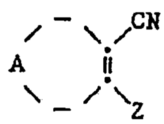

(IV)

wherein A is as defined above and Z is the radical of a carbonic acid derivative bonded via a nitrogen atom, with a hydrazide of the formula :

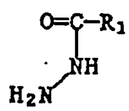

(V)

wherein $R_1$ is as defined above, or
d) to obtain a compound of formula I wherein $R_2$ represents hydrogen and X is oxygen, treating a compound of the formula :

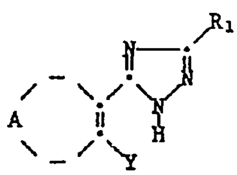

(VI),

wherein Y represents a group capable of being converted to the grouping –N=C=O by an oxidizing agent, with said oxidizing agent followed by ring closure, or
e) to obtain a compound of formula I, wherein $R_2$ is hydrogen and X is NH, converting a compound of the formula :

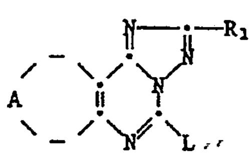

(VII),

wherein L is selected from halogen, $C_1$-$C_7$-alkoxy, phenyl-$C_1$-$C_7$-alkoxy, mercapto, $C_1$-$C_7$-alkylthio and isothiocyanato, by replacing the group L by the grouping NH, or
f) to obtain a compound of formula I wherein $R_2$ represents hydrogen, treating a compound of the formula :

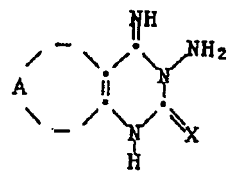

(VIII)

with a reactive derivative of a carboxylic acid of the formula $R_1$–COOH, and if desired, converting a resulting compound into another compound as defined above and/or converting a resulting salt into the free compound or into a different salt and/or converting a resulting free compound having a salt forming group into a salt.

2. A process according to claim 1 for the preparation of a tautomer of a compound of the formula Ia represented by the formulae :

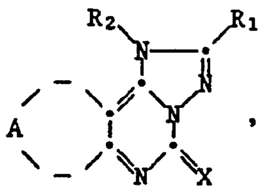

,

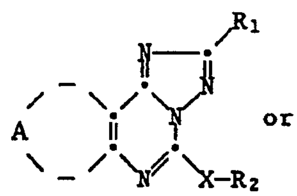

or

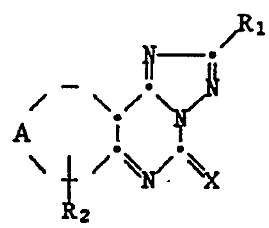

(Ib) (Ic) (Id)

characterised in that the process steps of claim 1 are carried out.

3. A process according to claims 1 or 2 for the preparation of a compound of the formulae Ia-Id, wherein X represents O, S, NH or NR ; R is $C_1$-$C_7$-alkyl, aryl-$C_1$-$C_7$-alkyl wherein aryl is phenyl, naphthyl or aromatic heteroaryl with 5-7 ring members having at least one heteroatom selected from O, N and S, but not more than one O or S atom, amino-$C_1$-$C_7$-alkyl, or $C_1$-$C_7$-alkylamino-$C_1$-$C_7$-alkyl ; $R_1$ represents phenyl or phenyl substituted by one to three groups selected from $C_1$-$C_7$-alkyl, $C_1$-$C_7$-alkoxy, hydroxy, halogen and trifluoromethyl ; or $R_1$ represents a heterocyclic radical bonded by way of a carbon atom, said heterocyclic radical being pyridyl, thienyl, furyl, pyrrolyl, thiazolyl, isothiazolyl, oxazolyl, imidazolyl, pyrazolyl, 1,2,3- or 1,2,4-triazolyl, tetrazolyl, pyrimidinyl, quinolyl, isoquinolyl, pyrrolinyl, pyrrolidinyl, dihydro-or tetrahydrofuranyl, dihydro- or tetrahydrothienyl, pyranyl, piperidinyl, morpholinyl, pyrazolinyl, thiazolinyl, oxazolinyl, triazolinyl, or ribofuranosyl, said heterocyclic group being unsubstituted or substituted by hydroxy, $C_1$-$C_7$-alkyl or halogen ; $R_2$ represents hydrogen, $C_1$-$C_7$-alkyl, aryl-$C_1$-$C_7$-alkyl, $C_2$-$C_7$-alkenyl, aryl-$C_2$-$C_7$-alkenyl or aryl wherein aryl is phenyl, naphthyl or aromatic heteroaryl with 5-7 ring members having at least one heteroatom selected from O, N and S, but not more than one O or S atom ; A is a bivalent bridging group of atoms selected from C, N, O, and S which forms together with the two adjacent carbon atoms to which A is attached a cyclohexene, cyclopentene, piperideine, tetrahydrobenzo[b]thiophene, pyridine, cycloheptene, dihydropyrrole, isoxazole, oxazole, isothiazole, thiazole, pyrazole, oxathiazole, dithiazole, pyrrole, furan, thiophene, oxazine, thiazine, pyridazine, pyran, thiopyran, oxathiin, dioxin, dithiin, pyrazine, pyrimidine or imidazole, a benzocondensated derivative thereof, or a partially saturated benzocondensated derivative thereof, these groups A being unsubstituted or substituted by $C_1$-$C_7$-alkyl, $C_1$-$C_7$ alkoxy, hydroxy, halogen, halo-$C_1$-$C_7$-alkyl, $C_1$-$C_7$-alkylthio, $C_1$-$C_7$alkylsulfonyl, $C_1$-$C_7$-alkylsulfinyl, aryl-$C_1$-$C_7$-alkyl wherein aryl is phenyl, naphthyl or aromatic heteroaryl with 5-7 ring members having at least one heteroatom selected from O, N and S, but not more than one O or S atom, amido, carbamoyl, $C_1$-$C_7$-alkoxycarbonyl or benzoyl ; and pharmaceutically acceptable salts thereof, characterised in that the process steps of claim 1 are carried out.

4. A process according to claims 1 or 2 for the preparation of a compound of the formulae Ia-Id, wherein X represents O, NH or NR wherein R represents $C_1$-$C_7$-alkyl ; $R_1$ represents phenyl or phenyl substituted by one to three groups selected from $C_1$-$C_7$-alkyl, $C_1$-$C_7$-alkoxy, hydroxy, halogen and trifluoromethyl ; or $R_1$ is 2- or 3-thienyl, 2- or 3-furyl, 2- or 3-pyrrolyl, 2-, 3- or 4-pyridyl, 3- or 4-pyrazolyl, or 2- or 4-imidazolyl, said heterocyclic radical being unsubstituted or substituted by hydroxy, $C_1$-$C_7$-alkyl or halogen ; $R_2$ represents hydrogen or $C_1$-$C_7$-alkyl ; and A is a bivalent bridging group of atoms selected from C, N, O and S

which forms together with the two adjacent carbon atoms to which A is attached a cyclohexene, cyclopentene, piperideine, tetrahydrobenzo[b]thiophene, pyridine, cycloheptene, dihydropyrrole, isoxazole, oxazole, isothiazole, thiazole, pyrazole, oxathiazole, dithiazole, pyrrole, furan, thiophene, oxazine, thiazine, pyridazine, pyran, thiopyran, oxathiin, dioxin, dithiin, pyrazine, pyrimidine or imidazole, a benzocondensated derivative thereof, or a partially saturated benzocondensated derivative thereof, these groups A being unsubstituted or substituted by $C_1$-$C_7$-alkyl, $C_1$-$C_7$-alkoxy, halo-$C_1$-$C_7$-alkyl, aryl-$C_1$-$C_7$-alkyl wherein aryl is phenyl, naphthyl or aromatic heteroaryl with 5-7 ring members having at least one heteroatom selected from O, N and S, but not more than one O or S atom, or $C_1$-$C_7$-alkoxycarbonyl ; and pharmaceutically acceptable salts thereof, characterised in that the process steps of claim 1 are carried out.

5. A process according to claims 1 or 2 for the preparation of a compound of the formulae Ia-Id, wherein X represents O or NH ; $R_1$ represents phenyl, phenyl substituted by halo in the ortho or meta position, or 2-furyl ; $R_2$ represents hydrogen ; and A is a bivalent bridging group of atoms selected from C, N, O, and S which forms together with the two adjacent carbon atoms to which A is attached a cyclohexene, cyclopentene, piperideine, tetrahydrobenzo[b]thiophene, pyridine, cycloheptene, dihydropyrrole, pyrazine, pyrimidine or imidazole, these groups A being unsubstituted or substituted by $C_1$-$C_7$-alkyl, $C_1$-$C_7$-alkoxy, halo-$C_1$-$C_7$alkyl, aryl-$C_1$-$C_7$-alkyl wherein aryl is phenyl, naphthyl or aromatic heteroaryl with 5-7 ring members having at least one heteroatom selected from O, N and S, but not more than one O or S atom, or $C_1$-$C_7$-alkoxycarbonyl ; and pharmaceutically acceptable salts thereof, characterised in that the process steps of claim 1 are carried out.

6. A process according to claim 1 for the preparation of a compound of the formula Ia wherein X is oxygen, $R_1$ is fluorophenyl and A is $-(CH_2)_5-$, and pharmaceutically acceptable salts thereof, characterised in that the process steps of claim 1 are carried out.

7. A process according to claim I for the preparation of a compound of the formula Ia wherein X is NH, $R_1$ is 2-furyl and A is $-(CH_2)_4-$, and pharmaceutically acceptable salts thereof, characterised in that the process steps of claim 1 are carried out.

8. A process according to claim 1 for the preparation of 9-Benzyl-2-phenyl-7,8,9,10-tetrahydropyrido[3,4-e][1,2,4]-triazolo-[1,5-c] pyrimidin-5(6H)one and pharmaceutically acceptable salts thereof, characterised in that the process steps of claim 1 are carried out.

9. A process according to claim 1 for the preparation of 9-Benzyl-2-(3-fluorophenyl)-7,8,9,10-tetrahydropyrido-[3,4-e][1,2,4]-triazolo-[1,5-c]-pyrimidine-5(6H)one and pharmaceutically acceptable salts thereof, characterised in that the process steps of claim 1 are carried out.

10. A process for the preparation of a pharmaceutical composition, characterised in that a compound of the formula Ia, its tautomers or pharmaceutically acceptable salts as defined in claim 1 is admixed with a pharmaceutically acceptable carrier.

## Ansprüche

### Patentansprüche für die Vertragsstaaten : BE, CH, DE, FR, GB, IT, LI, LU, NL, SE

1. Eine Verbindung der Formel

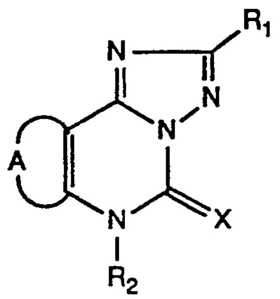

(Ia),

deren Tautomere und pharmazeutisch annehmbare Salze, worin

X O,S, NH oder NR bedeutet ;

R $C_1$-$C_7$-Alkyl, $C_2$-$C_7$-Alkenyl, $C_2$-$C_7$-Alkinyl, worin die ungesättigten Bindungen der Alkenyl- und Alkinylreste vom N-Atom in X durch wenigstens ein gesättigtes Kohlenstoffatom getrennt sind, $C_5$-$C_7$-Cycloalkyl oder Ringaromaten-$C_1$-$C_7$-Alkyl, worin der Ringaromat Phenyl, Naphthyl oder aromatisches Heteroaryl mit

5-7 Ringgliedern mit mindestens einem aus O, N und S ausgewählten Heteroatom, aber nicht mehr als einem O- oder S-Atom ist, wobei die oben genannten $C_1$-$C_7$-Alkylreste gegebenenfalls durch ein Heteroatom, ausgewählt aus O, N oder S, unterbrochen sind, bedeutet ; oder R Hydroxy, Hydroxy-$C_2$-$C_4$-Alkyl, Phenyl, Naphthyl oder aromatisches Heteroaryl mit 5-7 Ringgliedernmit mindestens einem aus O, N und S ausgewählten Heteroatom, aber nicht mehr als einem O- oder S-Atom ist, oder die Gruppe –C(=NH)–$NH_2$ bedeutet ;

$R_1$ Phenyl, Naphthyl, Pyridyl, Thienyl, Furyl, Pyrrolyl, Thiazolyl, Isothiazolyl, Oxazolyl, Imidazolyl, Pyrazolyl, 1,2,3- oder 1,2,4-Triazolyl, Tetrazolyl, Pyrimidinyl, Chinolyl, Isochinolyl, Pyrrolinyl, Pyrrolidinyl, Dihydro- oder Tetrahydrofuranyl, Dihydro- oder Tetrahydrothienyl, Pyranyl, Piperidinyl, Morpholinyl, Pyrazolinyl, Thiazolinyl, Oxazolinyl, Triazolinyl oder Ribofuranosyl ist, wobei diese Reste $R_1$ unsubstituiert oder durch Halogen, $C_1$-$C_7$-Alkyl, Halo-$C_1$-$C_7$-Alkyl, Hydroxy, $C_1$-$C_7$-Alkoxy, Hydroxy-$C_1$-$C_7$-Alkyl, Amino, Mono- oder Di-$C_1$-$C_7$ Alkylamino, $C_1$-$C_7$-Alkoxycarbonyl, Carbamoyl oder $C_1$-$C_7$-Alkylcarbamoyl subo stituiert sind, bedeutet ;

$R_2$ Wasserstoff, $C_1$-$C_7$-Alkyl, Hydroxy-$C_1$-$C_7$-Alkyl, $C_2$-$C_7$-Alkenyl, Aryl-$C_1$-$C_7$-Alkyl, Aryl-$C_2$-$C_7$-Alkenyl oder Aryl, wobei Aryl Phenyl, Naphthyl oder aromatisches Heteroaryl mit 5-7 Ringgliedern mit mindestens einem aus O, N und S ausgewählten Heteroatom, aber nicht mehr als einem O- oder S-Atom, bedeutet ;

A eine bivalente Überbrückungsgruppe mit aus C, O, N und S ausgewählten Atomen in der Kette, die zusammen mit den beiden anliegenden Kohlenstoffatomen, mit denen die Kette verknüpft ist, eine Cyclohexen-, Cyclopenten-, Piperidein-, Tetrahydrobenz[b]thiophen-, Pyridin-, Cyclohepten-, Dihydropyrrol-, Isoxazol-, Oxazol-, Isothiazol-, Thiazol-, Pyrazol-, Oxathiazol-, Dithiazol-, Pyrrol-, Furan-, Thiophen-, Oxazin-, Thiazin-, Pyridazin-, Pyran-, Thiopyran-, Oxathiin-, Dioxin-, Dithiin-, Pyrazin-, Pyrimidin- oder Imidazolgruppe, ein benzkondensiertes Derivat hiervon oder ein teilweise gesättigtes benzkondensiertes Derivat hiervon bildet, wobei diese Gruppen A unsubstituiert oder durch $C_1$-$C_7$-Alkyl, $C_1$-$C_7$-Alkoxy, Hydroxy, Halogen, Halo-$C_1$-$C_7$-Alkyl, Nitro, Amino, $C_1$-$C_7$-Alkylthio, $C_1$-$C_7$-Alkylsulfonyl, $C_1$-$C_7$-Alkylsulfinyl, Aryl-$C_1$-$C_7$-Alkyl, worin Aryl Phenyl, Naphthyl oder aromatisches Heteroaryl mit 5-7 Ringgliedern mit mindestens einem aus O, N und S ausgewählten Heteroatom, aber nicht mehr als einem O- oder S-Atom ist, Amido, Carbamoyl, $C_1$-$C_7$-Alkoxycarbonyl oder Benzoyl substituiert sind, bedeutet.

2. Ein Tautomeres einer Verbindung der Formel Ia nach Anspruch 1, dargestellt durch die Formeln

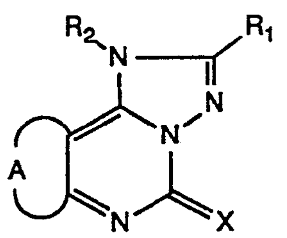

(Ib)

,

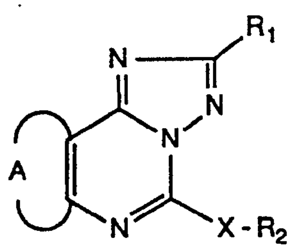

(Ic)

oder

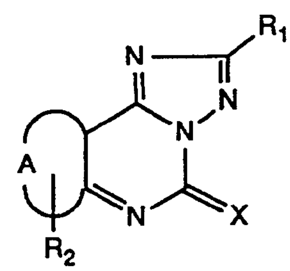

(Id)

3. Eine Verbindung nach Anspruch 1 oder 2 der Formeln Ia-Id, worin X O, S, NH oder NR bedeutet ; R $C_1$-$C_7$-Alkyl, Aryl-$C_1$-$C_7$-Alkyl, worin Aryl Phenyl, Naphthyl oder aromatisches Heteroaryl mit 5-7 Ringgliedern mit mindestens einem aus O, N und S ausgewählten Heteroatom, aber nicht mehr als einem O- oder S-Atom ist, Amino-$C_1$-$C_7$-Alkyl oder $C_1$-$C_7$-Alkylamino-$C_1$-$C_7$-Alkyl bedeutet ; $R_1$ Phenyl oder durch ein bis drei Reste ausgewählt aus $C_1$-$C_7$-Alkyl, $C_1$-$C_7$-Alkoxy, Hydroxy, Halogen und Trifluormethyl substituiertes Phenyl bedeutet ; oder $R_1$ ein über ein Kohlenstoffatom gebundenes heterocyclisches Radikal, wobei das besagte Radikal Pyridyl, Thienyl, Furyl, Pyrrolyl, Thiazolyl, Isothiazolyl, Oxazolyl, Imidazolyl, Pyrazolyl, 1,2,3- oder 1,2,4-Triazolyl, Tetrazolyl, Pyrimidinyl, Chinolyl, Isochinolinyl, Pyrrolinyl, Pyrrolidinyl, Dihydro- oder Tetrahydrofuranyl, Dihydro- oder Tetrahydrothienyl, Pyranyl, Piperidinyl, Morpholinyl, Pyrazolinyl, Thiazolinyl, Oxazolinyl, Triazolinyl oder Ribofuranosyl ist und die genannten heterocyclischen Gruppen unsubstituiert oder durch Hydroxy, $C_1$-$C_7$-Alkyl oder Halogen substituiert sind, bedeutet ; $R_2$ Wasserstoff, $C_1$-$C_7$-Alkyl, Aryl-$C_1$-$C_7$-Alkyl, $C_2$-$C_7$-Alkenyl, Aryl-$C_2$-$C_7$-Alkenyl oder Aryl, wobei Aryl Phenyl, Naphthyl oder aromatisches Heteroaryl mit 5-7 Ringgliedern mit mindestens einem aus O, N und S ausgewählten Heteroatom, aber nicht mehr als einem O- oder S-Atom, bedeutet ; A eine bivalente Überbrückungsgruppe mit aus C, N, O und S ausgewählten Atomen, die zusammen mit den beiden anliegenden Kohlenstoffatomen, mit denen A verbunden ist, eine Cyclohexen-, Cyclopenten-, Piperidein-, Tetrahydrobenz[b]thiophen-, Pyridin-, Cyclohepten-, Dihydropyrrol-, Isoxazol-, Oxazol-, Isothiazol-, Thiazol-, Pyrazol-, Oxathiazol-, Dithiazol-, Pyrrol-, Furan-, Thiophen-, Oxazin-, Thiazin-, Pyridazin-, Pyran-, Thiopyran-, Oxathiin-, Dioxin-,

Dithiin-, Pyrazin-, Pyrimidin- oder Imidazolgruppe, ein benzkondensiertes Derivat hiervon oder ein partiell gesättigtes benzkondensiertes Derivat hiervon bildet, wobei diese Gruppen A unsubstituiert oder durch $C_1$-$C_7$-Alkyl, $C_1$-$C_7$-Alkoxy, Hydroxy, Halogen, Halo-$C_1$-$C_7$-Alkyl, $C_1$-$C_7$-Alkylthio, $C_1$-$C_7$-Alkylsulfonyl, $C_1$-$C_7$-Alkylsulfinyl, Akyl-$C_1$-$C_7$-alkyl, worin Akyl Phenyl, Naphthyl oder aromatisches Heteroaryl mit 5-7 Ringgliedern mit mindestens einem aus O, N und S ausgewählten Heteroatom, aber nicht mehr als einem O- oder S-Atom ist, Amido, Carbamoyl, $C_1$-$C_7$-Alkoxycarbonyl oder Benzoyl substituiert sind, bedeutet; sowie deren pharmazeutisch annehmbare Salze.

4. Eine Verbindung nach Anspruch 1 oder 2 der Formeln Ia-Id, worin X O, NH oder NR, worin R $C_1$-$C_7$-Alkyl ist, bedeutet; $R_1$ Phenyl oder mit ein bis drei Resten ausgewählt aus $C_1$-$C_7$-Alkyl, $C_1$-$C_7$-Alkoxy, Hydroxy, Halogen und Trifluormethyl substituiertes Phenyl bedeutet; oder $R_1$ 2- oder 3-Thienyl, 2- oder 3-Furyl, 2- oder 3-Pyrrolyl, 2-, 3- oder 4-Pyridyl, 3- oder 4- Pyrazolyl, oder 2- oder 4-Imidazolyl, wobei die genannten heterocyclischen Reste unsubstituiert oder durch Hydroxy, $C_1$-$C_7$-Alkyl oder Halogen substituiert sind, bedeutet; $R_2$ Wasserstoff oder $C_1$-$C_7$-Alkyl bedeutet; und A eine bivalente Überbrückungsgruppe mit aus C, N, O und S ausgewählten Atomen, die zusammen mit den beiden anliegenden Kohlenstoffatomen, mit denen A verknüpft ist, eine Cyclohexen-, Cyclopenten-, Piperidein-, Tetrahydrobenz[b]thiophen-, Pyridin-, Cyclohepten-, Dihydropyrrol-, Isoxazol-, Oxazol-, Isothiazol-, Thiazol-, Pyrazol-, Oxathiazol-, Dithiazol-, Pyrrol-, Furan-, Thiophen-, Oxazin-, Thiazin-, Pyridazin-, Pyran-, Thiopyran-, Oxathiin-, Dioxin-, Dithiin-, Pyrazin-, Pyrimidin- oder Imidazolgruppe, ein benzkondensiertes Derivat hiervon oder ein partiell gesättigtes benzkondensiertes Derivat hiervon bilden, wobei diese Gruppen A unsubstituiert oder durch $C_1$-$C_7$-Alkyl, $C_1$-$C_7$-Alkoxy, Halo-$C_1$-$C_7$-Alkyl, Aryl-$C_1$-$C_7$-Alkyl, worin Aryl Phenyl, Naphthyl oder aromatisches Heteroaryl mit 5-7 Ringgliedern mit mindestens einem aus O, N und S ausgewählten Heteroatom, aber nicht mehr als einem O oder S-Atom ist, oder $C_1$-$C_7$-Alkoxycarbonyl substituiert sind, bedeutet; und deren pharmazeutisch annehmbare Salze.

5. Eine Verbindung nach Anspruch 1 oder 2 der Formeln Ia-Id, worin X O oder NH bedeutet; $R_1$ Phenyl, mit Halogen ortho- oder meta-substituiertes Phenyl oder 2-Furyl bedeutet; $R_2$ Wasserstoff bedeutet; und A eine bivalente Überbrückungsgruppe mit aus C, N, O und S ausgewählten Atomen, die mit den beiden anliegenden Kohlenstoffatomen, mit denen A verknüpft ist, eine Cyclohexen-, Cyclopenten-, Piperidein-, Tetrahydrobenz[b]thiophen-, Pyridin-, Cyclohepten-, Dihydropyrrol-, Pyrazin-, Pyrimidin- oder Imidazolgruppe bilden, wobei diese Gruppen A unsubstituiert oder durch $C_1$-$C_7$-Alkyl, $C_1$-$C_7$-Alkoxy, Halo-$C_1$-$C_7$-Alkyl, Aryl-$C_1$-$C_7$-Alkyl, worin Aryl Phenyl, Naphthyl oder aromatisches Heteroaryl mit 5-7 Ringgliedern mit mindestens einem aus O, N und S ausgewählten Heteroatom, aber nicht mehr als einem O- oder S-Atom ist, oder $C_1$-$C_7$-Alkoxycarbonyl substituiert sind, bedeutet; und deren pharmazeutisch annehmbare Salze.

6. Eine Verbindung nach Anspruch 1 der Formel Ia, worin X Sauerstoff, $R_1$ Fluorphenyl, und A $-(CH_2)_5-$ bedeutet, und deren pharmazeutisch annehmbare Salze.

7. Eine Verbindung nach Anspruch 1 der Formel Ia, worin X NH, $R_1$ 2-Furyl und A $-(CH_2)_4-$ bedeutet, und deren pharmazeutisch annehmbare Salze.

8. 9-Benzyl-2-phenyl-7,8,9,10-tetrahydropyrido[3,4-e][1,2,4]-triazolo[1,5-c]pyrimidin-5(6H)on und dessen pharmazeutisch annehmbare Salze nach Anspruch 1.

9. 9-Benzyl-2-(3-fluorphenyl)-7,8,9,10-tetrahydropyrido[3,4-e] [1,2,4]-triazolo[1,5-c]pyrimidin-5(6H)on und dessen pharmazeutisch annehmbare Salze nach Anspruch 1.

10. Eine pharmazeutische Zubereitung enthaltend eine Verbindung der Formel Ia, deren Tautomeren oder pharmazeutisch annehmbare Salze nach Anspruch 1 zusammen mit einem pharmazeutisch annehmbaren Trägermaterial.

11. Eine Verbindung nach einem der Ansprüche 1-9 zur Anwendung in einem Verfahren zur Behandlung von Menschen oder Tieren.

12. Eine Verbindung nach einem der Ansprüche 1-9 zur Anwendung als Anxiomodulator.

13. Eine Verbindung nach einem der Ansprüche 1-9 zur Anwendung als Benzdiazepin-Antagonist.

14. Verwendung einer Verbindung nach einem der Ansprüche 1-9 für die Herstellung einer pharmazeutischen Zubereitung.

15. Ein Verfahren zur Herstellung einer pharmazeutischen Zubereitung nach Anspruch 10, dadurch gekennzeichnet, dass eine Verbindung nach Anspruch 1 mit einem pharmazeutisch annehmbaren Trägermaterial gemischt wird.

16. Ein Verfahren zur Herstellung einer Verbindung nach Anspruch, 1 worin die Gruppen wie in Anspruch 1 definiert sind, dadurch gekennzeichnet, dass man

a) eine Verbindung der Formel

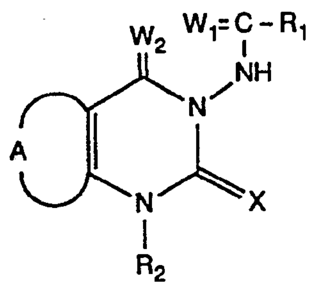

(II),

worin $R_1$, $R_2$, X und A wie oben definiert sind, einer der Reste $W_1$ und $W_2$ NH ist und der andere Rest $W_1$ oder $W_2$ O oder NH ist, durch Behandlung mit einer ein Stickstoffatom einführenden B ase cyclisiert, oder

b) eine Verbindung der Formel

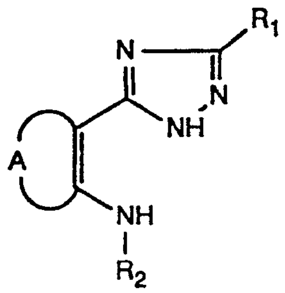

(III),

worin $R_1$, $R_2$ und A wie oben definiert sind, durch Behandlung mit einem reaktiven Derivat der Kohlensäure cyclisiert, oder

c) um eine Verbindung zu erhalten, worin $R_2$ Wasserstoff ist, eine Verbindung der Formel

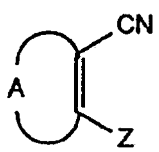

(IV),

worin A wie oben definiert ist und Z das Radikal eines Kohlensäuresäurederivates ist, das über ein Stickstoffatom gebunden ist, mit einem Hydrazid der Formel

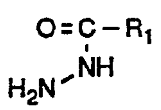

(V),

worin $R_1$ wie oben definiert ist, umsetzt, oder

d) um eine Verbindung der Formel I zu erhalten, worin $R_2$ Wasserstoff darstellt und X Sauerstoff ist, eine Verbindung der Formel

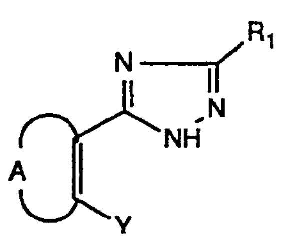

(VI),

worin Y eine Gruppe ist, die durch ein oxidierendes Agens in die Gruppe –N=C=O umgewandelt werden

kann, mit dem erwähnten oxidierenden Agens gefolgt von einem Ringschluss behandelt, oder
e) um eine Verbindung der Formel I zu erhalten, worin $R_2$ Wasserstolfff ist und X NH ist, eine Verbindung der Formel

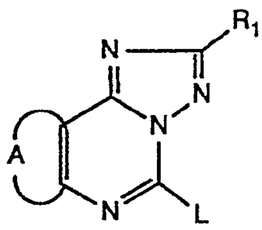

(VII)

worin L aus Halogen, $C_1$-$C_7$-Alkoxy, Phenyl-$C_1$-$C_7$-Alkoxy, Mercapto, $C_1$-$C_7$-Alkylthio und Isothiocyanato ausgewällt ist, unter Ersatz der Gruppe L durch die Gruppe NH umwandelt, oder
f) um eine Verbindung der Formel I zu erhalten, worin $R_2$ Wasserstoff ist, eine Verbindung der Formel

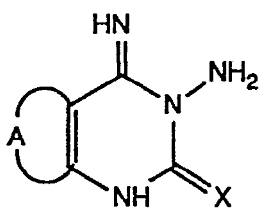

(VIII)

mit einem reaktiven Derivat einer Carbonsäure der Formel $R_1$–COOH behandelt und, falls erwünscht, eine erhaltene Verbindung in eine andere oben definierte Verbindung umwandelt und/oder ein erhaltenes Salz in die freie Verbindung oder in ein anderes Salz überführt und/oder eine erhaltene freie Verbindung mit einer salzbildenden Gruppe in ein Salz überführt.

**Patentansprüche für den Vertragsstaat : AT :**

1. Ein Verfalren zur Herstellung einer Verbindung der Formel

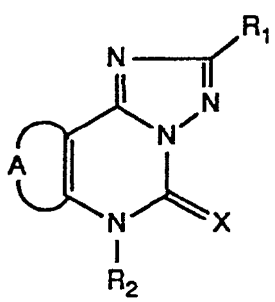

(Ia),

deren Tautomerer und pharmazeutisch annehmbarer Salze, worin

X O, S, NH oder NR bedeutet ;

R $C_1$-$C_7$-Alkyl, $C_2$-$C_7$-Alkenyl, $C_2$-$C_7$-Alkinyl, worin die ungesättigten Bindungen der Alkenyl- und Alkinylreste vom N-Atom in X durch wenigstens ein gesättigtes Kohlenstolfffatom getrennt sind, $C_5$-$C_7$-Cycloalkyl oder Ringaromaten-$C_1$-$C_7$-Alkyl, worin der Ringaromat Phenyl, Naphthyl oder aromatisches Heteroaryl mit 5-7 Ringgliedern mit mindestens einem aus O, N und S ausgewählten Heteroatom, aber nicht mehr als einem O- oder S-Atom ist, wobei die oben genannten $C_1$-$C_7$-Alkylreste gegebenenfalls durch ein Heteroatom ausgewällt aus O, N oder S unterbrochen sind, bedeutet ; oder R Hydroxy, Hydroxy-$C_2$-$C_4$-Alkyl, Phenyl, Naphthyl oder aromatisches Heteroaryl mit 5-7 Ringgliedern mit mindestens einem aus O, N und S ausgewählten Heteroatom, aber nicht mehr als einem O-oder S-Atom ist oder die Gruppe –C(=NH)–$NH_2$ bedeutet ;

$R_1$ Phenyl, Naphthyl, Pyridyl, Thienyl, Furyl, Pyrrolyl, Thiazolyl, Isothiazolyl, Oxazolyl, Imidazolyl, Pyrazolyl, 1,2,3- oder 1,2,4-Triazolyl, Tetrazolyl, Pyrimidinyl, Chinolyl, Isochinolyl, Pyrrolinyl, Pyrrolidinyl, Dihydro- oder Tetrahydrofuranyl, Dihydro- oder Tetrahydrothienyl, Pyranyl, Piperidinyl, Morpholinyl, Pyrazolinyl, Thiazolinyl, Oxazolinyl, Triazolinyl oder Ribofuranosyl, wobei diese Reste $R_1$ unsubstituiert oder durch Halogen, $C_1$-$C_7$-Alkyl, Halo-$C_1$-$C_7$-Alkyl, Hydroxy, $C_1$-$C_7$-Alkoxy, Hydroxy-$C_1$-$C_7$-Alkyl, Amino, Mono- oder Di-$C_1$-$C_7$-Alkylamino, $C_1$-$C_7$-Alkoxycarbonyl, Carbamoyl oder $C_1$-$C_7$-Alkylcarbamoyl substituiert sind, bedeutet ;

$R_2$ Wasserstolfff, $C_1$-$C_7$-Alkyl, Hydroxy-$C_1$-$C_7$-Alkyl, $C_2$-$C_7$-Alkenyl, Akyl-$C_1$-$C_7$-Alkyl, Akyl-$C_2$-$C_7$-Alkenyl oder Aryl, wobei Aryl Phenyl, Naphthyl oder aromatisches Heteroaryl mit 5-7 Ringgliedern ist mit mindestens einem aus O, N und S ausgewällten Heteroatom, aber nicht mehr als einem O- oder S-Atom, bedeutet ;

A eine bivalente Ueberbrückungsgruppe mit aus C, O, N und S ausgewählten Atomen in der Kette, die zusammen mit den beiden anliegenden Kohlenstoffatomen, mit denen die Kette verknüpft ist, eine Cyclohexen-, Cyclopenten-, Piperidein-, Tetrahydrobenz[b]thiophen-, Pyridin-, Cyclohepten-, Dihydropyrrol-, Isoxazol-, Oxazol-, Isothiazol-, Thiazol-, Pyrazol-, Oxathiazol-, Dithiazol-, Pyrrol-, Furan-, Thiophen-, Oxazin-, Thiazin-, Pyridazin-, Pyran-, Thiopyran-, Oxathiin-, Dioxin-, Dithiin-, Pyrazin-, Pyrimidin- oder Imidazolgruppe, ein benzkondensiertes Derivat hiervon, oder ein teilweise gesättigtes benzkondensiertes Derivat hiervon bildet, wobei diese Gruppen A unsubstituiert oder durch $C_1$-$C_7$-Alkyl, $C_1$-$C_7$-Alkoxy, Hydroxy, Halogen, Halo-$C_1$-$C_7$-Alkyl, Nitro, Amino, $C_1$-$C_7$-Alkylthio, $C_1$-$C_7$-Alkylsulfonyl, $C_1$-$C_7$-Alkylsulfinyl, Aryl-$C_1$-$C_7$-Alkyl, worin Aryl Phenyl, Naphthyl oder aromatisches Heteroaryl mit 5-7 Ringgliedern mit mindestens einem aus O, N oder S ausgewählten Heteroatom, aber nicht mehr als einem O- oder S-Atom ist, Amido, Carbamoyl, $C_1$-$C_7$-Alkoxycarbonyl oder Benzoyl sind, bedeutet, dadurch gekennzeichnet, dass man

a) eine Verbindung der Formel

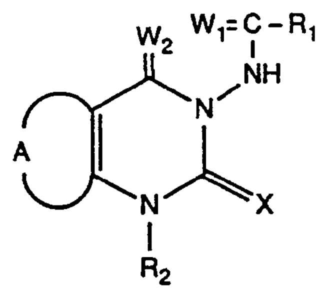

(II),

worin $R_1$, $R_2$, X und A wie oben definiert sind, einer der Reste $W_1$ und $W_2$ NH ist und der andere Rest $W_1$ oder $W_2$ O oder NH ist, durch Behandlung mit einer ein Stickstoffatom einführenden Base cyclisiert, oder

b) eine Verbindung der Formel

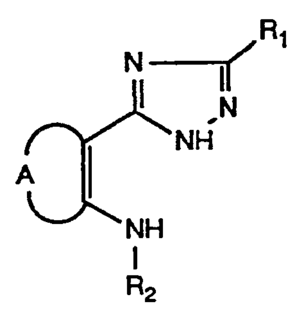

(III),

worin $R_1$, $R_2$ und A wie oben definiert sind, durch Behandlung mit einem reaktiven Derivat der Kohlensäure cyclisiert, oder

c) um eine Verbindung zu erhalten, worin R2 Wasserstoff ist, eine Verbindung der Formel

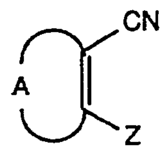

(IV),

worin A wie oben definiert ist und Z das Radikal eines Kohlensäurederivates ist, das über ein Stickstoffatom gebunden ist, mit einem Hydrazid der Formel

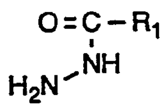

(V),

worin $R_1$ wie oben definiert ist, umsetzt, oder

d) um eine Verbindung der Formel I zu erhalten, worin $R_2$ Wasserstoff darstellt und X Sauerstoff ist, eine Verbindung der Formel

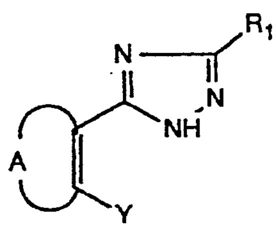

(VI),

worin Y eine Gruppe ist, die durch ein oxidierendes Agens in die Gruppe $-N=C=O$ umgewandelt werden kann, mit dem erwähnten oxidierenden Agens gefolgt von einem Ringschluss behandelt, oder

e) um eine Verbindung der Formel I zu erhalten, worin $R_2$ Wasserstoff ist und X NH ist, eine Verbindung der Formel

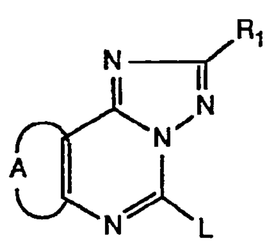

(VII)

worin L aus Halogen, $C_1$-$C_7$-Alkoxy, Phenyl-$C_1$-$C_7$-Alkoxy, Mercapto, $C_1$-$C_7$-Alkylthio und Isothiocyanato ausgewählt ist, unter Ersatz der Gruppe L durch die Gruppe NH umwandelt, oder

f) um eine Verbindung der Formel I zu erhalten, worin $R_2$ Wasserstoff ist, eine Verbindung der Formel

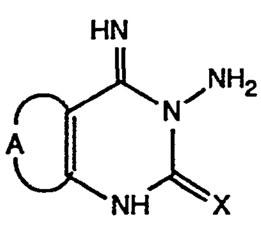

(VIII)

mit einem reaktiven Derivat einer Carbonsäure der Formel $R_1$–COOH behandelt und, falls erwünscht, eine erhaltene Verbindung in eine andere oben definierte Verbindung umwandelt und/oder ein erhaltenes Salz in die freie Verbindung oder in ein anderes Salz überführt und/oder eine erhaltene freie Verbindung mit einer salzbildenden Gruppe in ein Salz überführt.

2. Ein Verfalren nach Anspruch 1 zur Herstellung eines Tautomeren einer Verbindung der Formel Ia, dargestellt durch die Formeln

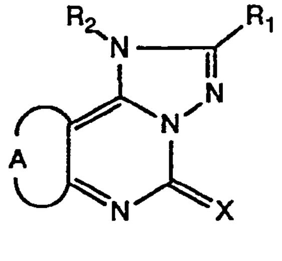

(Ib)

,

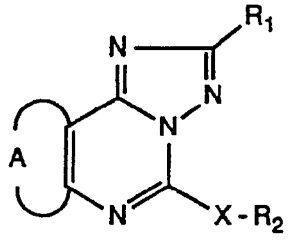

(Ic)

oder

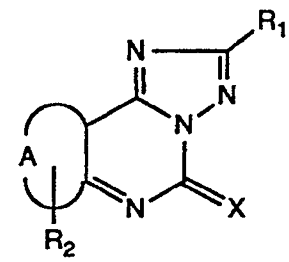

(Id),

dadurch gekennzeichnet, dass die Verfahrensschritte nach Anspruch 1 durchgeführt werden.

3. Ein Verfahren nach Anspruch 1 oder 2 zur Herstellung einer Verbindung der Formeln Ia-Id, worin X O, S, NH oder NR bedeutet ; R $C_1$-$C_7$-Alkyl, Aryl-$C_1$-$C_7$-Alkyl, worin Aryl Phenyl, Naphthyl oder aromatisches Heteroaryl mit 5-7 Ringgliedern mit mindestens einem aus O, N und S ausgewählten Heteroatom, aber nicht mehr als einem O- oder S-Atom ist, Amino-$C_1$-$C_7$-Alkyl Oder $C_1$-$C_7$-Alkylamino-$C_1$-$C_7$-Alkyl bedeutet ; $R_1$ Phenyl oder durch ein bis drei Reste ausgewählt aus $C_1$-$C_7$-Alkyl, $C_1$-$C_7$-Alkoxy, Hydroxy, Halogen und Trifluormethyl substituiertes Phenyl bedeutet ; oder $R_1$ ein über ein Kohlenstoffatom gebundenes heterocyclisches Radikal, wobei das besagte Radikal Pyridyl, Thienyl, Furyl, Pyrrolyl, Thiazolyl, Isothiazolyl, Oxazolyl, Imidazolyl, Pyrazolyl, 1,2,3- oder 1,2,4-Triazolyl, Tetrazolyl, Pyrimidinyl, Chinolyl, Isochinolinyl, Pyrrolinyl, Pyrrolidinyl, Dihydro- oder Tetrahydrofuranyl, Dihydro- oder Tetrahydrothienyl, Pyranyl, Piperidinyl, Morpholinyl, Pyrazolinyl, Thiazolinyl, Oxazolinyl, Triazolinyl oder Ribofwanosyl ist und die genannten heterocyclischen Gruppen unsubstituiert oder durch Hydroxy, $C_1$-$C_7$-Alkyl oder Halogen substituiert sind, bedeutet ; $R_2$ Wasserstoff, $C_1$-$C_7$-Alkyl, Aryl-$C_1$-$C_7$-Alkyl, $C_2$-$C_7$-Alkenyl, Aryl-$C_2$-$C_7$-Alkenyl oder Aryl, wobei Aryl Phenyl, Naphthyl oder aromatisches Heteroaryl mit 5-7 Ringgliedern mit mindestens einem aus O, N und S ausgewählten Heteroatom, aber nicht mehr als einem O- oder S-Atom, bedeutet ; A eine bivalente Überbrückungsgruppe mit aus C, N, O und S ausgewählten Atomen, die zusammen mit den beiden anliegenden Kohlenstoffatomen, mit denen A verbunden ist, eine Cyclohexen-, Cyclopenten-, Piperidein-, Tetralydrobenz[b]-thiophen-, Pyridin-, Cyclohepten-, Dihydropyrrol-, Isoxazol-, Oxazol-, Isothiazol-, Thiazol-, Pyrazol-, Oxathiazol-, Dithiazol-, Pyrrol-, Furan-, Thiophen-, Oxazin-, Thiazin-, Pyridazin-, Pyran-, Thiopyran-, Oxathiin-, Dioxin-, Dithiin-, Pyrazin-, Pyrimidin- oder Imidazolgruppe, ein benzkondensiertes Derivat hiervon oder ein partiell gesättigtes benzkondensiertes Derivat hiervon bildet, wobei diese Gruppen A unsubstituiert oder durch $C_1$-$C_7$-Alkyl, $C_1$-$C_7$-Alkoxy, Hydroxy, Halogen, Halo-$C_1$-$C_7$-Alkyl, $C_1$-$C_7$-Alkylthio, $C_1$-$C_7$-Alkylsulfonyl, $C_1$-$C_7$-Alkylsulfinyl, Aryl-$C_1$-$C_7$-alkyl, worin Aryl Phenyl, Naphthyl oder aromatisches Heteroaryl mit 5-7 Ringgliedern mit mindestens einem aus O, N und S ausgewählten Heteroatom, aber nicht mehr als einem O- oder S-Atom ist, Amido, Carbamoyl, $C_1$-$C_7$-Alkoxycarbonyl oder Benzoyl substituiert sind, bedeutet ; sowie deren pharmazeutisch annehmbarer Salze, dadurch gekennzeichnet, dass die Verfahrensschritte nach Anspruch 1 durchgeführt werden.

4. Ein Verfahren nach Anspruch 1 oder 2 zur Herstellung einer Verbindung der Formeln Ia-Id, worin X O, NH oder NR, worin R $C_1$-$C_7$-Alkyl ist, bedeutet ; $R_1$ Phenyl oder mit ein bis drei Resten ausgewählt aus $C_1$-$C_7$-Alkyl, $C_1$-$C_7$-Alkoxy, Hydroxy, Halogen und Trifluormethyl substituiertes Phenyl bedeutet ; oder $R_1$ 2- oder 3-Thienyl, 2- oder 3-Furyl, 2-oder 3-Poyrryl, 2-, 3- oder 4-Pyridyl, 3- oder 4- Pyrazolyl oder 2- oder 4-Imidazolyl, wobei die genannten heterocyclischen Reste unsubstituiert oder durch Hydroxy, $C_1$-$C_7$-Alkyl oder Halogen substituiert sind, bedeutet ; $R_2$ Wasserstoff oder $C_1$-$C_7$-Alkyl bedeutet ; und A eine bivalente Überbrückungsgruppe mit aus C, N, O und S ausgewählten Atomen, die zusammen mit den beiden anliegenden Kohlenstoffatomen, mit denen A verknüpft ist, eine Cyclohexen-, Cyclopenten-, Piperidein-, Tetrahydrobenz[b]thiophen-, Pyridin-, Cyclohepten-, Dihydropyrrol-, Isoxazol-, Oxazol-, Isothiazol-, Thiazol-, Pyrazol-, Oxathiazol-, Dithiazol-, Pyrrol-, Furan-, Thiophen-, Oxazin-, Thiazin-, Pyridazin-, Pyran-, Thiopyran-, Oxathiin-, Dioxin-, Dithiin-, Pyrazin-, Pyrimidin- oder Imidazolgruppe, ein benzkondensiertes Derivat hiervon oder ein partiell gesättigtes benzkondensiertes Derivat hiervon bilden, wobei diese Gruppen A unsubstituiert oder durch $C_1$-$C_7$-Alkyl, $C_1$-$C_7$ Alkoxy, Halo-$C_1$-$C_7$-Alkyl, Aryl-$C_1$-$C_7$-Alkyl, worin Aryl Phenyl,

Naphthyl oder aromatisches Heteroaryl mit 5-7 Ringgliedern mit mindestens einem aus O, N und S ausgewählten Heteroatom, aber nicht mehr als einem O- Oder S-Atom ist, oder $C_1$-$C_7$-Alkoxycarbonyl substituiert sind, bedeutet ; und deren pharmazeutisch annehmbarer Salze, dadurch gekennzeichnet, dass die Verfahrensschritte nach Anspruch 1 durchgeführt werden.

5. Ein Verfahren nach Anspruch 1 oder 2 zur Herstellung einer Verbindung der Formeln Ia-Id, worin X O- oder NH bedeutet ; $R_1$ Phenyl, mit Halogen ortho- oder meta-substituiertes Phenyl oder 2-Furyl bedeutet; $R_2$ Wasserstoff bedeutet ; und A eine bivalente Überbrückungsgruppe mit aus C, N, O und S ausgewählten Atomen, die mit den beiden anliegenden Kohlenstoffatomen, mit denen A verknüpft ist, eine Cyclohexen-, Cyclopenten-, Piperidein-, Tetrahydrobenz[b]thiophen-, Pyridin-, Cyclohepten-, Dihydropyrrol-, Pyrazin-, Pyrimidin- oder Imidazolgruppe bilden, wobei diese Gruppen A unsubstituiert oder durch $C_1$-$C_7$-Alkyl, $C_1$-$C_7$-Alkoxy, Halo-$C_1$-$C_7$-Alkyl, Aryl-$C_1$-$C_7$-Alkyl, worin Aryl Phenyl, Naphthyl oder aromatisches Heteroaryl mit 5-7 Ringgliedern mit mindestens einem aus O, N und S ausgewählten Heteroatom, aber nicht mehr als einem O- oder S-Atom ist, oder $C_1$-$C_7$-Alkoxycarbonyl substituiert sind, bedeutet ; und deren pharmazeutisch annehmbarer Salze, dadurch gekennzeichnet, dass die Verfahrensschritte nach Anspruch 1 durchgeführt werden.

6. Ein Verfahren nach Anspruch 1 zur Herstellung einer Verbindung der Formel Ia, worin X Sauerstoff, $R_1$ Fluorphenyl und A $-(CH_2)_5-$ bedeutet und deren pharmazeutisch annehmbarer Salze, dadurch gekennzeichnet, dass die Verfahrensschritte nach Anspruch 1 durchgeführt werden.

7. Ein Verfahren nach Anspruch 1 zur Herstellung einer Verbindung der Formel Ia, worin X NH, $R_1$ 2-Furyl und A $-(CH_2)_4-$ bedeutet und deren pharmazeutisch annehmbarer Salze, dadurch gekennzeichnet, dass die Verfalrensschritte nach Anspruch 1 durchgeführt werden.

8. Ein Verfahren nach Anspruch 1 zur Herstellung von 9-Benzyl-2-phenyl-7,8,9,10-tetrahydropyrido[3,4-e][1,2,4]-triazolo[1,5-c]pyrimidin-5(6H)on und dessen pharmazeutisch annehmbarer Salze, dadurch gekennzeichnet, dass die Verfahrensschritte nach Anspruch 1 durchgeführt werden.

9. Ein Verfahren nach Anspruch 1 zur Herstellung von 9-Benzyl-2-(3-fluorphenyl)7,8,9,10-tetrahydropyrido[3,4-e] [1,2,4]-triazolo[1,5-c]pyrimidin-5 (6H)on und dessen pharmazeutisch annehmbarer Salze, dadurch gekennzeichnet, dass die Verfahrensschritte nach Anspruch 1 durchgeführt werden.

10. Ein Verfahren zur Herstellung einer pharmazeutischen Zubereitung, dadurch gekennzeichnet, dass man eine Verbindung der Formel Ia, deren Tautomere oder pharmazeutisch annehmbare Salze, wie in Anspruch 1 definiert, mit einem pharmazeutisch annehmbaren Trägermaterial mischt.

## Revendications

## REVENDICATIONS pour les Etats contractants : BE, CH, DE, FR, GB, IT, LI, LU, NL, SE

1. Composé de formule :

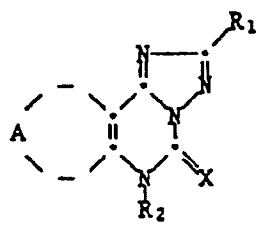

(Ia),

ses tautomères et sels pharmaceutiquement acceptables où

X représente O, S, NH ou NR ;

R représente un alcoyle en $C_{1-7}$, alcényle en $C_{2-7}$, alcynyle en $C_{2-7}$, où les liaisons non-saturées des groupes alcényle et alcynyle sont séparées des atomes de N de X par au moins un atome de carbone saturé, cycloalcoyle en $C_{5-7}$ ou noyau alcoyle en $C_{1-7}$ aromatique où le noyau aromatique est un phényle, naphtyle ou hétéroaryle aromatique avec 5 à 7 chaînons ayant au moins un hétéroatome choisi parmi O, N et S, mais pas plus d'un atome de O ou S, tous les groupes alcoyle en $C_{1-7}$ qui précèdent étant éventuellement interrompus par un hétéroatome choisi parmi O, N ou S ; ou R est un hydroxy, hydroxy-alcoyle en $C_{2-4}$, phényle, naphtyle ou hétéroaryle aromatique avec de 5 à 7 chaînons ayant au moins un hétéroatome choisi parmi O, N et S, mais pas plus d'un atome de O ou S, ou le groupe $-C(=NH)-NH_2$ ;

$R_1$ représente un phényle, naphtyle, pyridyle, thiényle, furyle, pyrrolyle, thiazolyle, isothiazolyle, oxazolyle, imidazolyle, pyrazolyle, 1,2,3- ou 1,2,4-triazolyle, tétrazolyle, pyrimidinyle, quinoléyle, isoquinoléyle, pyrrolinyle, pyrrolidinyle, dihydro- ou tétrahydrofuranyle, dihydro-ou tétrahydrothiényle, pyranyle, pipéridinyle, morpholinyle, pyrazolinyle, thiazolinyle, oxazolinyle, triazolinyle, ou ribofuranosyle, ces groupes $R_1$ étant non-substitués ou substitués par un halogène, alcoyle en $C_{1-7}$, halo-alcoyle en $C_{1-7}$, hydroxy, alcoxy en $C_{1-7}$, hydroxy-alcoyle en $C_{1-7}$, amino, mono- ou di-alcoyle en $C_{1-7}$-amino, alcoxy en $C_{1-7}$-carbonyle, carbamoyle ou alcoyle en $C_{1-7}$-carbamoyle ;

$R_2$ est un hydrogène, alcoyle en $C_{1-7}$, hydroxyalcoyle en $C_{1-7}$, alcényle en $C_{2-7}$, arylalcoyle en $C_{1-7}$, arylalcényle en $C_{2-7}$ ou aryle où aryle est un phényle, naphtyle ou hétéroaryle aromatique avec de 5 à 7 chainons ayant au moins un hétéroatome choisi parmi O, N et S, mais pas plus d'un atome de O ou S ;

A est un groupe de pontage bivalent contenant des atomes-chaînons choisis parmi C, O, N et S, qui forment avec les deux atomes de carbone adjacents auxquels la chaîne est attachée un groupe cyclohexène, cyclopentène, pipéridéine, tétrahydrobenzo[b]thiophène, pyridine, cycloheptène, dihydropyrrole, isoxazole, oxazole, isothiazole, thiazole, pyrazole, oxathiazole, dithiazole, pyrrole, furanne, thiophène, oxazine, thiazine, pyridazine, pyranne, thiopyranne, oxathiine, dioxine, dithiine, pyrazine, pyrimidine ou imidazole un de ses dérivés benzocondensés, ou un de ses dérivés benzocondensés partiellement saturé, ces groupes A étant non-substitués ou substitués par des groupes alcoyle en $C_{1-7}$, alcoxy en $C_{1-7}$, hydroxy,-halogène, halo-alcoyle en $C_{1-7}$, nitro, amino, alcoythio en $C_{1-7}$, alcoyle en $C_{1-7}$-sulfonyle, alcoyle en $C_{1-7}$-sulfinyle, aryl-alcoyle en $C_{1-7}$ où aryle est un phényle, naphtyle ou hétéroaryle aromatique avec de 5 à 7 chaînons ayant au moins un hétéroatome choisi parmi O, N et S, mais pas plus d'un atome de O ou S, amido, carbamoyle, alcoxy en $C_{1-7}$-carbonyle ou benzoyle.

2. Tautomère d'un composé de formule Ia selon la revendication 1, représenté par les formules :

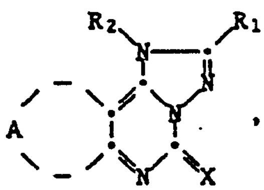 ,

(Ib)

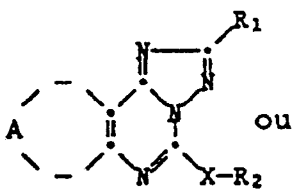 ou

(Ic)

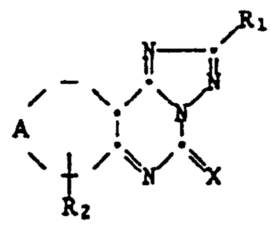

(Id)

3. Composé selon les revendications 1 ou 2 de formules Ia-Id où X représente O, S, NH ou NR ; R est un alcoyle en $C_{1-7}$, aryl-alcoyle en $C_{1-7}$ où aryle est un phényle, naphtyle, ou hétéroaryle aromatique avec 5 à 7 chaînons ayant au moins un hétéroatome choisi parmi O, N et S, mais pas plus d'un atome de O ou S, un amino-alcoyle en $C_{1-7}$, ou alcoylamino en $C_{1-7}$-alcoyle en $C_{1-7}$ ; $R_1$ représente un phényle ou phényle substitué par de un à trois groupes choisis parmi alcoyle en $C_{1-7}$, alcoxy en $C_{1-7}$, hydroxy, halogène et trifluorométhyle ; ou $R_1$ représente un radical hétérocyclique lié par un atome de carbone, ledit radical hétérocyclique étant un pyridyle, thiényle, furyle, pyrrolyle, thiazolyle, isothiazolyle, oxazolyle, imidazolyle, pyrazolyle, 1,2,3- ou 1,2,4-triazolyle, tétrazolyle, pyrimidinyle, quinoléyle, iscquinoléyle, pyrrolinyle, pyrrolidinyle, dihydroou tétrahydrofuranyle, dihydro-ou tétrahydrothiényle, pyranyle, pipéridinyle, morpholinyle, pyrazolinyle, thiazolinyle, oxazolinyle, triazolinyle, ou ribofuranosyle, ledit groupe hétérocyclique étant non-substitué ou substitué par un hydroxy, alcoyle en $C_{1-7}$ ou halogène ; $R_2$ représente un hydrogène, alcoyle en $C_{1-7}$, aryl-alcoyle en $C_{1-7}$, alcényle en $C_{2-7}$, aryl-alcényle en $C_{2-7}$ ou aryle où aryle est un phényle, naphtyle ou hétéroaryle aromatique avec de 5 à 7 chaînons ayant au moins un hétéroatome choisi parmi O, N et S, mais pas plus d'un atome de O ou de S ; A est un groupe de pontage bivalent d'atomes choisis parmi C, N, O et S qui forme ensemble avec les deux atomes de carbone adjacents auxquels A est attaché un cyclohexène, cyclopentène, pipéridéine, tétrahydrobenzo[b]thiophène, pyridine, cycloheptène, dihydropyrrole, isoxazole, oxazole, isothiazole, thiazole, pyrazole, oxathiazole, dithiazole, pyrrole, furanne, thiophène, oxazine, thiazine, pyridazine, pyranne, thiopyranne, oxathiine, dioxine, dithiine, pyrazine, pyrimidine ou imidazole un de leurs dérivés benzocondensés, ou un de leurs dérivés benzocondensés partiellement saturés, ces groupes A étant non-substitués ou substitués par un alcoyle en $C_{1-7}$, alcoxy en $C_{1-7}$, hydroxy, halogène, halo-alcoyle en $C_{1-7}$, alcoylthio en $C_{1-7}$ alcoyle en $C_{1-7}$-sulfonyle, alcoyle en $C_{1-7}$-sulfinyle, aryl-alcoyle en $C_{1-7}$ où aryle est un phényle, naphtyle ou hétéroaryle aromatique avec de 5 à 7 chaînons ayant au moins un hétéroatome choisi parmi O, N et S, mais pas plus d'un atome de O ou S, amido, carbamoyle, alcoxy en $C_{1-7}$-carbonyle, ou benzoyle ; et leurs sels pharmaceutiquement acceptables.

4. Composé selon les revendications 1 ou 2 des formules Ia-Id, où X représente O, NH ou Nr où R repré-

sente un alcoyle en $C_{1-7}$ ; $R_1$ représente un phényle ou phényle substitué par un à trois groupes choisis parmi alcoyle en $C_{1-7}$, alcoxy en $C_{1-7}$, hydroxy, halogène, et trifluorométhyle ; ou $R_1$ est un 2- ou 3-thiényle, 2- ou 3-furyle, 2- ou 3-pyrrolyle, 2, 3- ou 4-pyridyle, 3- ou 4-pyrazolyle, ou 2- ou 4-imidazolyle, ledit radical hétérocyclique étant non-substitué ou substitué par un hydroxy, alcoyle en $C_{1-7}$ ou halogène ; $R_2$ représente un hydrogène ou alcoyle en $C_{1-7}$ ; et A est un groupe de pontage bivalent d'atomes choisis parmi C, N, O et S qui forment ensemble avec les deux atomes de carbone adjacents auxquels A est attaché un cyclohexène, cyclopentène, pipéridéine, tétrahydrobenzo[b]thiophène, pyridine, cycloheptène, dihydropyrrole, isoxazole, oxazole, isothiazole, thiazole, pyrazole, oxathiazole, diothiazole, pyrrole, furanne, thiophène, oxazine, thiazine, pyridazine, pyranne, thiopyranne, oxathiine, dioxine, dithiine, pyrazine, pyrimidine ou imidazole, un de leurs dérivés benzocondensés ou un de leurs dérivés benzocondensés partiellement saturés ces groupes A étant non-substitués ou substitués par un alcoyle en $C_{1-7}$, alcoxy en $C_{1-7}$, halo-alcoyle en $C_{1-7}$, aryl-alcoyle en $C_{1-7}$ où aryle est un phényle, naphtyle ou hétéroaryle aromatique avec 5 à 7 chaînons ayant au moins un hétéroatome choisi parmi O, N et S, mais pas plus d'un atome de O ou S, ou alcoxy en $C_{1-7}$-carbonyle ; et ses sels pharmaceutiquement acceptables.

5. Composé selon les revendications 1 ou 2 de formules Ia-Id, où X représente o ou NH ; $R_1$ représente un phényle, phényle substitué par un halo en position ortho ou méta, ou un 2-furyle ; $R_2$ représente un hydrogène, et A est un groupe de pontage bivalent d'atomes choisis parmi C, N, O et S qui forme ensemble avec les deux atomes de carbone adjacents auxquels A est attaché un cyclohexène, cyclopentène, pipéridéine, tétrahydrobenzo|b|thiophène, pyridine, cycloheptène, dihydropyrrole, pyrazine, pyrimidine ou imidazole, ces groupes A étant non-substitués ou substitués par un alcoyle en $C_{1-7}$, alcoxy en $C_{1-7}$, halo-alcoyle en $C_{1-7}$, aryl-alcoyle en $C_{1-7}$ où aryle est un phényle, naphtyle ou hétéroaryle aromatique avec 5 à 7 chaînons ayant au moins un hétéroatome choisi parmi O, N et S, mais par plus d'un atome de O ou S, ou alcoxy en $C_{1-7}$-carbonyle ; et ses sels pharmaceutiquement acceptables.

6. Composé selon la revendication 1 deformule Ia où X est un oxygène, $R_1$ est un fluorophényle et A est un $-(CH_2)_5-$, et ses sels pharmaceutiquement acceptables.

7. Composé selon la revendication 1, de formule Ia, où X représente NH, $R_1$ est un 2-furyle et A est un $-(CH_2)_4-$ et ses sels pharmaceutiquement acceptables.

8. 9-benzyl-2-phényl-7,8,9,10-tétrahydropyrido[3,4-e][1,2,4]-triazolo[1,5-c]pyrimidin-5(6H)one et ses sels pharmaceutiquement acceptables selon la revendication 1.

9.9-benzyl-2-(3-fluorophényl)-7,8,9,10-tétrahydropyrido[3,4-e]-[1,2,4]triazolo[1,5-c]pyrimidine-5(6H) one et ses sels pharmaceutiquement acceptables selon la revendication 1.

10. Composition pharmaceutique contenant un composé de formule Ia, ses tautomères ou sels pharmaceutiquement acceptables selon la revendication 1 ainsi qu'un support pharmaceutiquement acceptable.

11. Composé selon les revendications 19 pour application dans le traitement de l'homme ou de l'animal.

12. Composé selon les revendications 19 pour application comme anxiomodulateur.

13. Composé selon les revendications 19 pour application comme antagoniste de la benzodiazépine.

14. Application d'un composés selon les revendications 1-9 pour la préparation d'une composition pharmaceutique.

15. Procédé de préparation d'une composition pharmaceutique selon la revendication 10, caractérisé en ce qu'on mélange un composé selon la revendication 1 avec un support pharmaceutiquement acceptable.

16. Procédé de préparation d'un composé selon la revendication 1 où les groupes sont tels que définis dans la revendication 1, qui comprend

a) cyclisation d'un composé de formule :

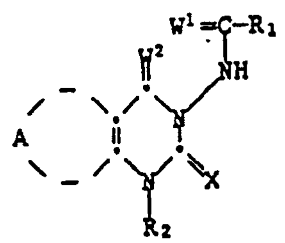

(II),

où $R_1$, $R_2$, X et A sont tels que définis ci-dessus, l'un des radicaux $W^1$ et $W^2$ représente NH et l'autre représente O ou NH, par traitement avec une base fournissant un atome d'azote, ou

b) cyclisation d'un composé de formule :

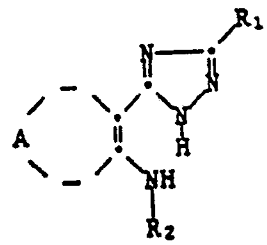

(III),

où $R_1$, $R_2$ et A sont tels que définis ci-dessus, par traitement avec un dérivé réactif de l'acide carbonique, ou

c) pour obtenir un composé où $R_2$ est un hydrogène, réaction d'un composé de formule :

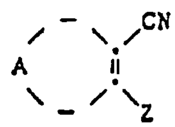

(IV)

où A est tel que défini ci-dessus et Z est le radical d'un dérivé d'acide carbonique lié via un atome d'azote, avec un hydrazide de formule :

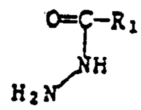

(V)

où $R_1$ est tel que défini ci-dessus, ou

d) pour obtenir un composé de formule I où $R_2$ représente un hydrogène et X un oxygène, traitement d'un composé de formule

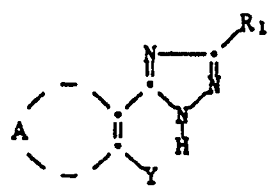

(VI),

où Y représente un groupe capable d'être transformé en le groupement $-N=C=O$ par un agent oxydant, ledit agent oxydant étant suivi d'une cyclisation, ou

e) pour obtenir un composé de formule I, où $R_2$ est un hydrogène et X représente NH, transformation d'un composé de formule :

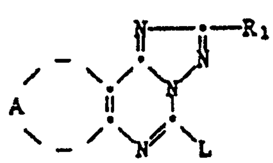

(VII),

où L est choisi parmi halogène, alcoxy en $C_{1-7}$, phénylalcoxy en $C_{1-7}$, mercapto, alcoylthio en $C_{1-7}$ et isothiocyanato, en remplaçant le groupe L par le groupement NH, ou

f) pour obtenir un composé de formule I où $R_2$ représente un hydrogène, traitement d'un composé de formule :

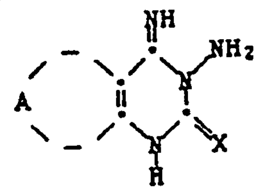

(VIII)

avec un dérivé réactif d'un acide carboxylique de formule $R_1$–COH, et, si on le désire, transformation d'un composé résultant en un autre composé tel que défini ci-dessus et/ou transformation d'un sel résultant en le composé libre ou en un sel différent et/ou transformation d'un composé libre résultant ayant un groupe salificateur en un sel.

**REVENDICATIONS pour l'Etat contractant : AT**

1. Procédé de préparation d'un composé de formule

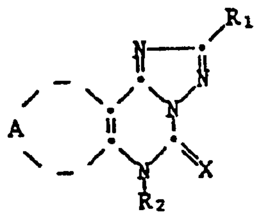

(Ia)

ses tautomères et sels pharmaceutiquement acceptables où

X représente O, S, NH ou NR ;

R représente un alcoyle en $C_{1-7}$, alcényle en $C_{2-7}$, alcynyle en $C_{2-7}$, où les liaisons non-saturées des groupes alcényle et alcynyle sont séparées des atomes de N de X par au moins un atome de carbone saturé, cycloalcoyle en $C_{5-7}$ ou noyau alcoyle en $C_{1-7}$ aromatique où le noyau aromatique est un phényle, naphtyle ou hétéroaryle aromatique avec 5 à 7 chaînons ayant au moins un hétéroatome choisi parmi O, N et S, mais pas plus d'un atome de O ou S, tous les groupes alcoyle en $C_{1-7}$ qui précèdent étant éventuellement interrompus par un hétéroatome choisi parmi O, N ou S ; ou R est un hydroxy, hydroxy-alcoyle en $C_{2-4}$, phényle, naphtyle ou hétéroaryle aromatique avec de 5 à 7 chaînons ayant au moins un hétéroatome choisi parmi O, N et S, mais pas plus d'un atome de O ou S, ou le groupe $-C(=NH)-NH_2$,

$R_1$ représente un phényle, naphtyle, pyridyle, thiényle, furyle, pyrrolyle, thiazolyle, isothiazolyle, oxazolyle, imidazolyle, pyrazolyle, 1,2,3- ou 1,2,4-triazolyle, tétrazolyle, pyrimidinyle, quinoléyle, isoquinoléyle, pyrrolinyle, pyrrolidinyle, dihydro- ou tétrahydrofuranyle, dihydro-ou tétrahydrothiényle, pyranyle, pipéridinyle, morpholinyle, pyrazolinyle, thiazolinyle, oxazolinyle, triazolinyle, ou ribofuranosyle, ces groupes $R_1$ étant non-substitués ou substitués par un halogène, alcoyle en $C_{1-7}$, halo-alcoyle en $C_{1-7}$, hydroxy, alcoxy en $C_{1-7}$, hydroxy-alcoyle en $C_{1-7}$, amino, mono- ou di-alcoyle en $C_{1-7}$-amino, alcoxy en $C_{1-7}$-carbonyle, carbamoyle ou alcoyle en $C_{1-7}$-carbamoyle ;

$R_2$ est un hydrogène, alcoyle en $C_{1-7}$, hydroxy-alcoyle en $C_{1-7}$, alcényle en $C_{2-7}$, aryl-alcoyle en $C_{1-7}$, aryl-alcényle en $C_{2-7}$ ou aryle où aryle est un phényle, naphtyle ou hétéroaryle aromatique avec de 5 à 7 chaînons ayant au moins un hétéroatome choisi parmi O, N et S, mais pas plus d'un atome de O ou S ;

A est un groupe de pontage bivalent contenant des atomes-chaînons choisis parmi C, O, N et S, qui forment avec les deux atomes de carbone adjacents auxquels la chaîne est attachée un groupe cyclohexène, cyclopentène, pipéridéine, tétrahydrobenzo[b]thiophène, pyridine, cycloheptène, dihydropyrrole, isoxazole, oxazole, isothiazole, thiazole, pyrazole, oxathiazole, dithiazole, pyrrole, furanne, thiophène, oxazine, thiazine, pyridazine, pyranne, thiopyranne, oxathiine, dioxine, dithiine, pyrazine, pyrimidine ou imidazole un de ses dérivés benzocondensés, ou un de ses derivés benzocondensés partiellement saturé, ces groupes A étant non-substitués ou substitués par des groupes alcoyle en $C_{1-7}$, alcoxy en $C_{1-7}$, hydroxy, halogène, halo-alcoyle en $C_{1-7}$, nitro, amino, alcoythio en $C_{1-7}$, alcoyle en $C_{1-7}$-sulfonyle, alcoyle en $C_{1-7}$-sulfinyle, aryl-alcoyle en $C_{1-7}$ où aryle est un phényle, naphtyle ou hetéroaryle aromatique avec de 5 à 7 chaînons ayant au moins un hetéroatome choisi parmi O, N et S, mais pas plus d'un atome de O ou S, amido, carbamoyle, alcoxy en $C_{1-7}$-carbonyle ou benzoyle qui comprend

a) cyclisation d'un composé de formule :

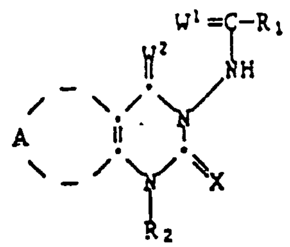

(II),

où $R_1$, $R_2$, X et A sont tels que définis ci-dessus, l'un des radicaux $W^1$ et $W^2$ représente NH et l'autre représente O ou NH, par traitement avec une base fournissant un atome d'azote, ou

b) cyclisation d'un composé de formule :

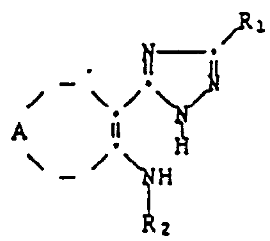

(III),

où $R_1$, $R_2$ et A sont tels que définis ci-dessus, par traitement avec un dérive réactif de l'acide carbonique, ou

c) pour obtenir un composé où $R_2$ est un hydrogène, réaction d'un composé de formule :

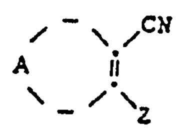

(IV)

où A est tel que defini ci-dessus et Z est le radical d'un dérivé d'acide carbonique lié via un atome d'azote, avec un hydrazide de formule :

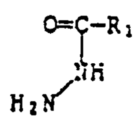

(V)

où $R_1$ est tel que défini ci-dessus, ou

d) pour obtenir un composé de formule I où $R_2$ représente un hydrogène et X un oxygène, traitement d'un composé de formule

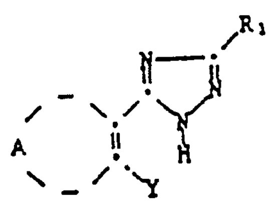

(VI),

où Y représente un groupe capable d'être transformé en le groupement $-N=C=O$ par un agent oxydant, ledit agent oxydant étant suivi d'une cyclisation,

ou

e) pour obtenir un composé de formule I, où $R_2$ est un hydrogène et X représente NH, transformation d'un composé de formule :

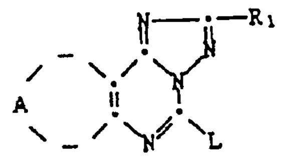

(VII),

où L est choisi parmi halogène, alcoxy en $C_{1-7}$, phényl-alcoxy en $C_{1-7}$, mercapto, alcoylthio en $C_{1-7}$ et isothiocyanato, en remplaçant le groupe L par le groupement NH, ou

f) pour obtenir un composé de formule I où $R_2$ représente un hydrogène, traitement d'un compose de formule :

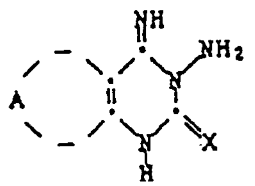

(VIII)

avec un dérivé réactif d'un acide carboxylique de formule $R_1$–CO H, et, si on le désire, transformation d'un composé résultant en un autre composé tel que défini ci-dessus et/ou transformation d'un sel résultant en le compose libre ou en un sel différent et/ou transformation d'un composé libre résultant ayant un groupe salificateur en un sel, caractérisé en ce qu'on effectue les étapes du procédé revendication 1.

2. Procédé selon la revendication 1 pour la préparation d'un tautomère d'un composé de formule Ia représenté par les formules :

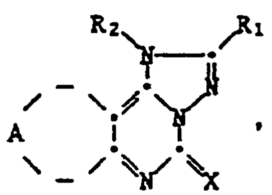

,

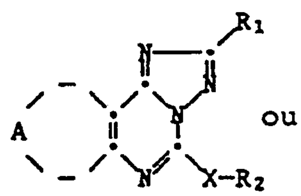

ou

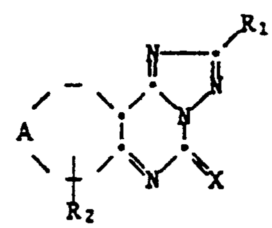

(Ib) (Ic) (Id)

3. Procédé selon les revendications 1 ou 2 de préparation d'un composé de formules Ia-Id où X représente O, S, NH ou NR, R est un alcoyle en $C_{1-7}$, aryl-alcoyle en $C_{1-7}$ où aryle est un phényle, naphtyle, ou hétéroaryle aromatique avec 5 à 7 chaînons ayant au moins un hétéroatome choisi parmi O, N et S, mais pas plus d'un atome de O ou S, un amino-alcoyle en $C_{1-7}$, ou alcoylamino en $C_{1-7}$-alcoyle en $C_{1-7}$ ; $R_1$ représente un phényle ou phényle substitué par de un à trois groupes choisis parmi alcoyle en $C_{1-7}$, alcoxy en $C_{1-7}$, hydroxy, halogène et trifluorométhyle ; ou $R_1$ représente un radical hétérocyclique lié par un atome de carbone, ledit radical hétérocyclique étant un pyridyle, thiényle, furyle, pyrrolyle, thiazolyle, isothiazolyle, oxazolyle, imidazolyle, pyrazolyle, 1,2,3- ou 1,2,4-triazolyle, tétrazolyle, pyrimidinyle, quinoléyle, isoquinoléyle, pyrrolinyle, pyrrolidinyle, dihydro-ou tétrahydrofuranyle, dihydro- ou tétrahydrothiényle, pyranyle, pipéridinyle, morpholinyle, pyrazolinyle, thiazolinyle, oxazolinyle, triazolinyle, ou ribofuranosyle, ledit groupe hétérocyclique étant non-substitué ou substitué par un hydroxy, alcoyle en $C_{1-7}$ ou halogène ; $R_2$ représente un hydrogène, alcoyle en $C_{1-7}$, aryl-alcoyle en $C_{1-7}$, alcényle en $C_{2-7}$, aryl-alcényle en $C_{2-7}$ ou aryle où aryle est un phényle, naphtyle ou hétéroaryle aromatique avec de 5 à 7 chaînons ayant au moins un hétéroatome choisi parmi O, N et S, mais pas plus d'un atome de O ou de S ; A est un groupe de pontage bivalent d'atomes choisis parmi C, N, O et S qui forme ensemble avec les deux atomes de carbone adjacents auxquels A est attaché un cyclohexène, cyclopentène, pipéridéine, tétrahydrobenzo[b]thiophène, pyridine, cycloheptène, dihydropyrrole, isoxazole, oxazole, isothiazole, thiazole, pyrazole, oxathiazole, dithiazole, pyrrole, furanne, thiophène, oxazine, thiazine, pyridazine, pyranne, thiopyranne, oxathiine, dioxine, dithiine, pyrazine, pyrimidine ou imidazole un de leurs dérivés benzocondensés, ou un de leurs dérivés benzocondensés partiellement saturés, ces groupes A étant non-substitués ou substitués par un

alcoyle en $C_{1-7}$, alcoxy en $C_{1-7}$, hydroxy, halogène, halo-alcoyle en $C_{1-7}$, alcoylthio en $C_{1-7}$ alcoyle en $C_{1-7}$-sulfonyle, alcoyle en $C_{1-7}$-sulfinyle, aryl-alcoyle en $C_{1-7}$ où aryle est un phényle, naphtyle ou hétéroaryle aromatique avec de 5 à 7 chaînons ayant au moins un hétéroatome choisi parmi O, N et S, mais pas plus d'un atome de O ou S, amido, carbamoyle, alcoxy en $C_{1-7}$-carbonyle, ou benzoyle ; et de ses sels pharmaceutiquement acceptables, caractérisé en ce qu'on effectue les étapes du procédé de la revendication 1.

4. Procédé selon les revendications 1 ou 2 de préparation d'un composé de formules Ia-Id, où X représente O, NH ou NR où R représente un alcoyle en $C_{1-7}$ ; $R_1$ représente un phényle ou phényle substitué par un à trois groupes choisis parmi alcoyle en $C_{1-7}$, alcoxy en $C_{1-7}$, hydroxy, halogène, et trifluorométhyle; ou $R_1$ est un 2- ou 3-thiényle, 2- ou 3-furyle, 2- ou 3-pyrrolyle, 2, 3- ou 4-pyridyle, 3- ou 4-pyrazolyle, ou 2- ou 4-imidazolyle, ledit radical hétérocyclique étant non-substitué ou substitué par un hydroxy, alcoyle en $C_{1-7}$ ou halogène ; $R_2$ représente un hydrogène ou alcoyle en $C_{1-7}$ ; et A est un groupe de pontage bivalent d'atomes choisis parmi C, N, O et S qui forment ensemble avec les deux atomes de carbone adjacents auxquels A est attaché un cyclohexène, cyclopentène, pipéridéine, tétrahydrobenzo[b]thiophène, pyridine, cycloheptène, dihydropyrrole, isoxazole, oxazole, isothiazole, thiazole, pyrazole, oxathiazole, dicthiazole, pyrrole, furanne, thiophène, oxazine, thiazine, pyridazine, pyranne, thiopyranne, oxathiine, dioxine, dithiine, pyrazine, pyrimidine ou imidazole, un de leurs dérivés benzocondensés ou un de leurs dérivés benzocondensés partiellement saturés ces groupes A étant non-substitués ou substitués par un alcoyle en $C_{1-7}$, alcoxy en $C_{1-7}$, halo-alcoyle en $C_{1-7}$, aryl-alcoyle en $C_{1-7}$ où aryle est un phényle, naphtyle ou héteroaryle aromatique avec 5 à 7 chaînons ayant au moins un hétéroatome choisi parmi O, N et S, mais pas plus d'un atome de O ou S, ou alcoxy en $C_{1-7}$-carbonyle ; et de ses sels pharmaceutiquement acceptables, caractérisé en ce qu'on effectue les étapes du procédé de revendication 1.

5. Procédé selon les revendications 1 ou 2 de préparation d'un composé de formules Ia-Id, où X représente O ou NH ; $R_1$ représente un phényle, phényle substitué par un halo en position ortho ou méta, ou un 2-furyle ; $R_2$ représente un hydrogène, et A est un groupe de pontage bivalent d'atomes choisis parmi C, N, O et S qui forme ensemble avec les deux atomes de carbone adjacents auxquels A est attaché un cyclohexène, cyclopentène, pipéridéine, tétrahydrobenzo[b]thiophène, pyridine, cycloheptène, dihydropyrrole, pyrazine, pyrimidine ou imidazole, ces groupes A étant non-substitués ou substitués par un alcoyle en $C_{1-7}$, alcoxy en $C_{1-7}$, halo-alcoyle en $C_{1-7}$, aryl-alcoyle en $C_{1-7}$ où aryle est un phényle, naphtyle ou hétéroaryle aromatique avec 5 à 7 chaînons ayant au moins un hétéroatome choisi parmi O, N et S, mais par plus d'un atome de O ou S, ou alcoxy en $C_{1-7}$-carbonyle ; et de ses sels pharmaceutiquement acceptables, caractérisé en ce qu'on effectue les étapes du procédé de la revendication 1.

6. Procédé selon la revendication 1, de préparation d'un composé de formule Ia où X est un oxygène, $R_1$ est un fluorophényle et A représente $-(CH_2)_5-$, et de ses sels pharmaceutiquement acceptables, caractérisé en ce qu'on effectue les étapes du procédé de la revendication 1.

7. Procédé selon la revendication 1 de préparation d'un composé de formule Ia où X représente NH, $R_1$ est un 2-furyle et A représente $-(CH_2)_4-$, et de ses sels pharmaceutiquement acceptables, caractérisé en ce qu'on effectue les étapes du procédé de la revendication 1.

8. Procédé selon la revendication 1, de préparation de la 9-benzyl-2-phényl-7,8,9,10-tétrahydropyrido[3,4-e][1,2,4]-triazolo[1,5-c]-pyrimidin-5(6H)one et de ses sels pharmaceutiquement acceptables, caractérisé en ce qu'on effectue les étapes du procédé de la revendication 1.

9. Procédé selon la revendication 1 de préparation de la 9-benzyl-2-(3-fluorophényl)-7,8,9,10-tétrahydropyrido[3,4-e][1,2,4]-triazolo[1,5-c]pyrimidine-5(6H)one et de ses sels pharmaceutiquement acceptables, caractérisé en ce qu'on effectue les étapes du procédé de la revendication 1.

10. Procédé de préparation d'une composition pharmaceutique, caractérisé en ce qu'on mélange un composé de formule Ia, ses tautomères ou sels pharmaceutiquement acceptables tels que définis dans la revendication 1, avec un support pharmaceutiquement acceptable.